# EUROPEAN PATENT SPECIFICATION

(11) **EP 0 414 475 B1**
(45) Date of publication and mention of the grant of the patent: **10.12.1997**
(21) Application number: 90309120.5
(22) Date of filing: 21.08.1990
(51) Int. Cl.: C12N 5/06, C07K 14/18, C12N 7/00, G01N 33/576

(54) **Methods for culturing HCV in B- or T-lymphocyte cell lines**
Verfahren zur HCV-Züchtung in Zell-Linien aus B- oder T-Lymphozyten
Procédé de culture du HCV dans des lignées cellulaire dérivée des lymphocytes-B ou -T

(30) Priority: 25.08.1989 US 398667
(43) Date of publication of application: 27.02.1991
(73) Proprietor: CHIRON CORPORATION, Emeryville, California 94608 (US)
(72) Inventor: Weiner, Amy J., Oakland, California 94611 (US); Steimer, Kathelyn S., Benicia, California 94510 (US); Houghton, Michael, Danville, CA 94526 (US)
(74) Representative: Goldin, Douglas Michael

(56) References cited:
- EP-A- 0 318 216
- WO-A-82/00205
- WO-A-82/02774
- WO-A-87/05930

### SEQUENCE LISTING

### (1) GENERAL INFORMATION:

(i) APPLICANT:
   (A) NAME: Chiron Corporation
   (B) STREET: 4560 Horton Street
   (C) CITY: Emeryville
   (D) STATE: California
   (E) COUNTRY: USA
   (F) POSTAL CODE (ZIP): 94608
(ii) TITLE OF INVENTION: Methods for Culturing HCV in Eukaryotic Cells
(iii) NUMBER OF SEQUENCES: 5
(v) CURRENT APPLICATION DATA:
   APPLICATION NUMBER: EP 90309120.5

### (2) INFORMATION FOR SEQ ID NO:1:

(i) SEQUENCE CHARACTERISTICS:
   (A) LENGTH: 16 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:1:
   GCATGTCATG ATGTAT 16

### (2) INFORMATION FOR SEQ ID NO:2:

(i) SEQUENCE CHARACTERISTICS:
   (A) LENGTH: 16 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:2:
   ACAATACGTG TGTCAC 16

### (2) INFORMATION FOR SEQ ID NO:3:

(i) SEQUENCE CHARACTERISTICS:
   (A) LENGTH: 9185 base pairs
   (B) TYPE: nucleic acid
   (C) STRANDEDNESS: double
   (D) TOPOLOGY: linear
(ii) MOLECULE TYPE: cDNA
(vi) ORIGINAL SOURCE:
   (A) ORGANISM: Hepatitis C virus
   (B) STRAIN: HCV1
(xi) SEQUENCE DESCRIPTION: SEQ ID NO:3:
   CACTCCACCA TGAATCACTC CCCTGTGAGG AACTACTGTC TTCACGCAGA AAGCGTCTAG      60
   CCATGGCGTT AGTATGAGTG TCGTGCAGCC TCCAGGACCC CCCCTCCCGG GAGAGCCATA     120
   GTGGTCTGCG GAACCGGTGA GTACACCGGA ATTGCCAGGA CGACCGGGTC CTTTCTTGGA     180
   TCAACCCGCT CAATGCCTGG AGATTTGGGC GTGCCCCCGC AAGACTGCTA GCCGAGTAGT     240
   GTTGGGTCGC GAAAGGCCTT GTGGTACTGC CTGATAGGGT GCTTGCGAGT GCCCCGGGAG     300
   GTCTCGTAGA CCGTGCACCA TGAGCACGAA TCCTAAACCT CAAAAAAAAA ACAAACGTAA     360
   CACCAACCGT CGCCCACAGG ACGTCAAGTT CCCGGGTGGC GGTCAGATCG TTGGTGGAGT     420
   TTACTTGTTG CCGCGCAGGG GCCCTAGATT GGGTGTGCGC GCGACGAGAA AGACTTCCGA     480
   GCGGTCGCAA CCTCGAGGTA GACGTCAGCC TATCCCCAAG GCTCGTCGGC CCGAGGGCAG     540
   GACCTGGGCT CAGCCCGGGT ACCCTTGGCC CCTCTATGGC AATGAGGGCT GCGGGTGGGC     600
   GGGATGGCTC CTGTCTCCCC GTGGCTCTCG GCCTAGCTGG GGCCCCACAG ACCCCCGGCG     660
   TAGGTCGCGC AATTTGGGTA AGGTCATCGA TACCCTTACG TGCGGCTTCG CCGACCTCAT     720
   GGGGTACATA CCGCTCGTCG GCGCCCCTCT TGGAGGCGCT GCCAGGGCCC TGGCGCATGG 780
   CGTCCGGGTT CTGGAAGACG GCGTGAACTA TGCAACAGGG AACCTTCCTG GTTGCTCTTT 840
   CTCTATCTTC CTTCTGGCCC TGCTCTCTTG CTTGACTGTG CCCGCTTCGG CCTACCAAGT 900
   GCGCAACTCC ACGGGGCTTT ACCACGTCAC CAATGATTGC CCTAACTCGA GTATTGTGTA 960
   CGAGGCGGCC GATGCCATCC TGCACACTCC GGGGTGCGTC CCTTGCGTTC GTGAGGGCAA 1020
   CGCCTCGAGG TGTTGGGTGG CGATGACCCC TACGGTGGCC ACCAGGGATG GCAAACTCCC 1080
   CGCGACGCAG CTTCGACGTC ACATCGATCT GCTTGTCGGG AGCGCCACCC TCTGTTCGGC 1140
   CCTCTACGTG GGGGACCTAT GCGGGTCTGT CTTTCTTGTC GGCCAACTGT TCACCTTCTC 1200
   TCCCAGGCGC CACTGGACGA CGCAAGGTTG CAATTGCTCT ATCTATCCCG GCCATATAAC 1260
   GGGTCACCGC ATGGCATGGG ATATGATGAT GAACTGGTCC CCTACGACGG CGTTGGTAAT 1320
   GGCTCAGCTG CTCCGGATCC CACAAGCCAT CTTGGACATG ATCGCTGGTG CTCACTGGGG 1380
   AGTCCTGGCG GGCATAGCGT ATTTCTCCAT GGTGGGGAAC TGGGCGAAGG TCCTGGTAGT 1440
   GCTGCTGCTA TTTGCCGGCG TCGACGCGGA AACCCACGTC ACCGGGGGAA GTGCCGGCCA 1500
   CACTGTGTCT GGATTTGTTA GCCTCCTCGC ACCAGGCGCC AAGCAGAACG TCCAGCTGAT 1560
   CAACACCAAC GGCAGTTGGC ACCTCAATAG CACGGCCCTG AACTGCAATG ATAGCCTCAA 1620
   CACCGGCTGG TTGGCAGGGC TTTTCTATCA CCACAAGTTC AACTCTTCAG GCTGTCCTGA 1680
   GAGGCTAGCC AGCTGCCGAC CCCTTACCGA TTTTGACCAG GGCTGGGGCC CTATCAGTTA 1740
   TGCCAACGGA AGCGGCCCCG ACCAGCGCCC CTACTGCTGG CACTACCCCC CAAAACCTTG 1800
   CGGTATTGTG CCCGCGAAGA GTGTGTGTGG TCCGGTATAT TGCTTCACTC CCAGCCCCGT 1860
   GGTGGTGGGA ACGACCGACA GGTCGGGCGC GCCCACCTAC AGCTGGGGTG AAAATGATAC 1920
   GGACGTCTTC GTCCTTAACA ATACCAGGCC ACCGCTGGGC AATTGGTTCG GTTGTACCTG 1980
   GATGAACTCA ACTGGATTCA CCAAAGTGTG CGGAGCGCCT CCTTGTGTCA TCGGAGGGGC 2040
   GGGCAACAAC ACCCTGCACT GCCCCACTGA TTGCTTCCGC AAGCATCCGG ACGCCACATA 2100
   CTCTCGGTGC GGCTCCGGTC CCTGGATCAC ACCCAGGTGC CTGGTCGACT ACCCGTATAG 2160
   GCTTTGGCAT TATCCTTGTA CCATCAACTA CACCATATTT AAAATCAGGA TGTACGTGGG 2220
   AGGGGTCGAA CACAGGCTGG AAGCTGCCTG CAACTGGACG CGGGGCGAAC GTTGCGATCT 2280
   GGAAGACAGG GACAGGTCCG AGCTCAGCCC GTTACTGCTG ACCACTACAC AGTGGCAGGT 2340
   CCTCCCGTGT TCCTTCACAA CCCTACCAGC CTTGTCCACC GGCCTCATCC ACCTCCACCA 2400
   GAACATTGTG GACGTGCAGT ACTTGTACGG GGTGGGGTCA AGCATCGCGT CCTGGGCCAT 2460
   TAAGTGGGAG TACGTCGTTC TCCTGTTCCT TCTGCTTGCA GACGCGCGCG TCTGCTCCTG 2520
   CTTGTGGATG ATGCTACTCA TATCCCAAGC GGAGGCGGCT TTGGAGAACC TCGTAATACT 2580
   TAATGCAGCA TCCCTGGCCG GGACGCACGG TCTTGTATCC TTCCTCGTGT TCTTCTGCTT 2640
   TGCATGGTAT TTGAAGGGTA AGTGGGTGCC CGGAGCGGTC TACACCTTCT ACGGGATGTG 2700
   GCCTCTCCTC CTGCTCCTGT TGGCGTTGCC CCAGCGGGCG TACGCGCTGG ACACGGAGGT 2760
   GGCCGCGTCG TGTGGCGGTG TTGTTCTCGT CGGGTTGATG GCGCTGACTC TGTCACCATA 2820
   TTACAAGCGC TATATCAGCT GGTGCTTGTG GTGGCTTCAG TATTTTCTGA CCAGAGTGGA 2880
   AGCGCAACTG CACGTGTGGA TTCCCCCCCT CAACGTCCGA GGGGGGCGCG ACGCCGTCAT 2940
   CTTACTCATG TGTGCTGTAC ACCCGACTCT GGTATTTGAC ATCACCAAAT TGCTGCTGGC 3000
   CGTCTTCGGA CCCCTTTGGA TTCTTCAAGC CAGTTTGCTT AAAGTACCCT ACTTTGTGCG 3060
   CGTCCAAGGC CTTCTCCGGT TCTGCGCGTT AGCGCGGAAG ATGATCGGAG GCCATTACGT 3120
   GCAAATGGTC ATCATTAAGT TAGGGGCGCT TACTGGCACC TATGTTTATA ACCATCTCAC 3180
   TCCTCTTCGG GACTGGGCGC ACAACGGCTT GCGAGATCTG GCCGTGGCTG TAGAGCCAGT 3240
   CGTCTTCTCC CAAATGGAGA CCAAGCTCAT CACGTGGGGG GCAGATACCG CCGCGTGCGG 3300
   TGACATCATC AACGGCTTGC CTGTTTCCGC CCGCAGGGGC CGGGAGATAC TGCTCGGGCC 3360
   AGCCGATGGA ATGGTCTCCA AGGGGTGGAG GTTGCTGGCG CCCATCACGG CGTACGCCCA 3420
   GCAGACAAGG GGCCTCCTAG GGTGCATAAT CACCAGCCTA ACTGGCCGGG ACAAAAACCA 3480
   AGTGGAGGGT GAGGTCCAGA TTGTGTCAAC TGCTGCCCAA ACCTTCCTGG CAACGTGCAT 3540
   CAATGGGGTG TGCTGGACTG TCTACCACGG GGCCGGAACG AGGACCATCG CGTCACCCAA 3600
   GGGTCCTGTC ATCCAGATGT ATACCAATGT AGACCAAGAC CTTGTGGGCT GGCCCGCTCC 3660
   GCAAGGTAGC CGCTCATTGA CACCCTGCAC TTGCGGCTCC TCGGACCTTT ACCTGGTCAC 3720
   GAGGCACGCC GATGTCATTC CCGTGCGCCG GCGGGGTGAT AGCAGGGGCA GCCTGCTGTC 3780
   GCCCCGGCCC ATTTCCTACT TGAAAGGCTC CTCGGGGGGT CCGCTGTTGT GCCCCGCGGG 3840
   GCACGCCGTG GGCATATTTA GGGCCGCGGT GTGCACCCGT GGAGTGGCTA AGGCGGTGGA 3900
   CTTTATCCCT GTGGAGAACC TAGAGACAAC CATGAGGTCC CCGGTGTTCA CGGATAACTC 3960
   CTCTCCACCA GTAGTGCCCC AGAGCTTCCA GGTGGCTCAC CTCCATGCTC CCACAGGCAG 4020
   CGGCAAAAGC ACCAAGGTCC CGGCTGCATA TGCAGCTCAG GGCTATAAGG TGCTAGTACT 4080
   CAACCCCTCT GTTGCTGCAA CACTGGGCTT TGGTGCTTAC ATGTCCAAGG CTCATGGGAT 4140
   CGATCCTAAC ATCAGGACCG GGGTGAGAAC AATTACCACT GGCAGCCCCA TCACGTACTC 4200
   CACCTACGGC AAGTTCCTTG CCGACGGCGG GTGCTCGGGG GGCGCTTATG ACATAATAAT 4260
   TTGTGACGAG TGCCACTCCA CGGATGCCAC ATCCATCTTG GGCATCGGCA CTGTCCTTGA 4320
   CCAAGCAGAG ACTGCGGGGG CGAGACTGGT TGTGCTCGCC ACCGCCACCC CTCCGGGCTC 4380
   CGTCACTGTG CCCCATCCCA ACATCGAGGA GGTTGCTCTG TCCACCACCG GAGAGATCCC 4440
   TTTTTACGGC AAGGCTATCC CCCTCGAAGT AATCAAGGGG GGGAGACATC TCATCTTCTG 4500
   TCATTCAAAG AAGAAGTGCG ACGAACTCGC CGCAAAGCTG GTCGCATTGG GCATCAATGC 4560
   CGTGGCCTAC TACCGCGGTC TTGACGTGTC CGTCATCCCG ACCAGCGGCG ATGTTGTCGT 4620
   CGTGGCAACC GATGCCCTCA TGACCGGCTA TACCGGCGAC TTCGACTCGG TGATAGACTG 4680
   CAATACGTGT GTCACCCAGA CAGTCGATTT CAGCCTTGAC CCTACCTTCA CCATTGAGAC 4740
   AATCACGCTC CCCCAGGATG CTGTCTCCCG CACTCAACGT CGGGGCAGGA CTGGCAGGGG 4800
   GAAGCCAGGC ATCTACAGAT TTGTGGCACC GGGGGAGCGC CCCTCCGGCA TGTTCGACTC 4860
   GTCCGTCCTC TGTGAGTGCT ATGACGCAGG CTGTGCTTGG TATGAGCTCA CGCCCGCCGA 4920
   GACTACAGTT AGGCTACGAG CGTACATGAA CACCCCGGGG CTTCCCGTGT GCCAGGACCA 4980
   TCTTGAATTT TGGGAGGGCG TCTTTACAGG CCTCACTCAT ATAGATGCCC ACTTTCTATC 5040
   CCAGACAAAG CAGAGTGGGG AGAACCTTCC TTACCTGGTA GCGTACCAAG CCACCGTGTG 5100
   CGCTAGGGCT CAAGCCCCTC CCCCATCGTG GGACCAGATG TGGAAGTGTT TGATTCGCCT 5160
   CAAGCCCACC CTCCATGGGC CAACACCCCT GCTATACAGA CTGGGCGCTG TTCAGAATGA 5220
   AATCACCCTG ACGCACCCAG TCACCAAATA CATCATGACA TGCATGTCGG CCGACCTGGA 5280
   GGTCGTCACG AGCACCTGGG TGCTCGTTGG CGGCGTCCTG GCTGCTTTGG CCGCGTATTG 5340
   CCTGTCAACA GGCTGCGTGG TCATAGTGGG CAGGGTCGTC TTGTCCGGGA AGCCGGCAAT 5400
   CATACCTGAC AGGGAAGTCC TCTACCGAGA GTTCGATGAG ATGGAAGAGT GCTCTCAGCA 5460
   CTTACCGTAC ATCGAGCAAG GGATGATGCT CGCCGAGCAG TTCAAGCAGA AGGCCCTCGG 5520
   CCTCCTGCAG ACCGCGTCCC GTCAGGCAGA GGTTATCGCC CCTGCTGTCC AGACCAACTG 5580
   GCAAAAACTC GAGACCTTCT GGGCGAAGCA TATGTGGAAC TTCATCAGTG GGATACAATA 5640
   CTTGGCGGGC TTGTCAACGC TGCCTGGTAA CCCCGCCATT GCTTCATTGA TGGCTTTTAC 5700
   AGCTGCTGTC ACCAGCCCAC TAACCACTAG CCAAACCCTC CTCTTCAACA TATTGGGGGG 5760
   GTGGGTGGCT GCCCAGCTCG CCGCCCCCGG TGCCGCTACT GCCTTTGTGG GCGCTGGCTT 5820
   AGCTGGCGCC GCCATCGGCA GTGTTGGACT GGGGAAGGTC CTCATAGACA TCCTTGCAGG 5880
   GTATGGCGCG GGCGTGGCGG GAGCTCTTGT GGCATTCAAG ATCATGAGCG GTGAGGTCCC 5940
   CTCCACGGAG GACCTGGTCA ATCTACTGCC CGCCATCCTC TCGCCCGGAG CCCTCGTAGT 6000
   CGGCGTGGTC TGTGCAGCAA TACTGCGCCG GCACGTTGGC CCGGGCGAGG GGGCAGTGCA 6060
   GTGGATGAAC CGGCTGATAG CCTTCGCCTC CCGGGGGAAC CATGTTTCCC CCACGCACTA 6120
   CGTGCCGGAG AGCGATGCAG CTGCCCGCGT CACTGCCATA CTCAGCAGCC TCACTGTAAC 6180
   CCAGCTCCTG AGGCGACTGC ACCAGTGGAT AAGCTCGGAG TGTACCACTC CATGCTCCGG 6240
   TTCCTGGCTA AGGGACATCT GGGACTGGAT ATGCGAGGTG TTGAGCGACT TTAAGACCTG 6300
   GCTAAAAGCT AAGCTCATGC CACAGCTGCC TGGGATCCCC TTTGTGTCCT GCCAGCGCGG 6360
   GTATAAGGGG GTCTGGCGAG TGGACGGCAT CATGCACACT CGCTGCCACT GTGGAGCTGA 6420
   GATCACTGGA CATGTCAAAA ACGGGACGAT GAGGATCGTC GGTCCTAGGA CCTGCAGGAA 6480
   CATGTGGAGT GGGACCTTCC CCATTAATGC CTACACCACG GGCCCCTGTA CCCCCCTTCC 6540
   TGCGCCGAAC TACACGTTCG CGCTATGGAG GGTGTCTGCA GAGGAATATG TGGAGATAAG 6600
   GCAGGTGGGG GACTTCCACT ACGTGACGGG TATGACTACT GACAATCTCA AATGCCCGTG 6660
   CCAGGTCCCA TCGCCCGAAT TTTTCACAGA ATTGGACGGG GTGCGCCTAC ATAGGTTTGC 6720
   GCCCCCCTGC AAGCCCTTGC TGCGGGAGGA GGTATCATTC AGAGTAGGAC TCCACGAATA 6780
   CCCGGTAGGG TCGCAATTAC CTTGCGAGCC CGAACCGGAC GTGGCCGTGT TGACGTCCAT 6840
   GCTCACTGAT CCCTCCCATA TAACAGCAGA GGCGGCCGGG CGAAGGTTGG CGAGGGGATC 6900
   ACCCCCCTCT GTGGCCAGCT CCTCGGCTAG CCAGCTATCC GCTCCATCTC TCAAGGCAAC 6960
   TTGCACCGCT AACCATGACT CCCCTGATGC TGAGCTCATA GAGGCCAACC TCCTATGGAG 7020
   GCAGGAGATG GGCGGCAACA TCACCAGGGT TGAGTCAGAA AACAAAGTGG TGATTCTGGA 7080
   CTCCTTCGAT CCGCTTGTGG CGGAGGAGGA CGAGCGGGAG ATCTCCGTAC CCGCAGAAAT 7140
   CCTGCGGAAG TCTCGGAGAT TCGCCCAGGC CCTGCCCGTT TGGGCGCGGC CGGACTATAA 7200
   CCCCCCGCTA GTGGAGACGT GGAAAAAGCC CGACTACGAA CCACCTGTGG TCCATGGCTG 7260
   TCCGCTTCCA CCTCCAAAGT CCCCTCCTGT GCCTCCGCCT CGGAAGAAGC GGACGGTGGT 7320
   CCTCACTGAA TCAACCCTAT CTACTGCCTT GGCCGAGCTC GCCACCAGAA GCTTTGGCAG 7380
   CTCCTCAACT TCCGGCATTA CGGGCGACAA TACGACAACA TCCTCTGAGC CCGCCCCTTC 7440
   TGGCTGCCCC CCCGACTCCG ACGCTGAGTC CTATTCCTCC ATGCCCCCCC TGGAGGGGGA 7500
   GCCTGGGGAT CCGGATCTTA GCGACGGGTC ATGGTCAACG GTCAGTAGTG AGGCCAACGC 7560
   GGAGGATGTC GTGTGCTGCT CAATGTCTTA CTCTTGGACA GGCGCACTCG TCACCCCGTG 7620
   CGCCGCGGAA GAACAGAAAC TGCCCATCAA TGCACTAAGC AACTCGTTGC TACGTCACCA 7680
   CAATTTGGTG TATTCCACCA CCTCACGCAG TGCTTGCCAA AGGCAGAAGA AAGTCACATT 7740
   TGACAGACTG CAAGTTCTGG ACAGCCATTA CCAGGACGTA CTCAAGGAGG TTAAAGCAGC 7800
   GGCGTCAAAA GTGAAGGCTA ACTTGCTATC CGTAGAGGAA GCTTGCAGCC TGACGCCCCC 7860
   ACACTCAGCC AAATCCAAGT TTGGTTATGG GGCAAAAGAC GTCCGTTGCC ATGCCAGAAA 7920
   GGCCGTAACC CACATCAACT CCGTGTGGAA AGACCTTCTG GAAGACAATG TAACACCAAT 7980
   AGACACTACC ATCATGGCTA AGAACGAGGT TTTCTGCGTT CAGCCTGAGA AGGGGGGTCG 8040
   TAAGCCAGCT CGTCTCATCG TGTTCCCCGA TCTGGGCGTG CGCGTGTGCG AAAAGATGGC 8100
   TTTGTACGAC GTGGTTACAA AGCTCCCCTT GGCCGTGATG GGAAGCTCCT ACGGATTCCA 8160
   ATACTCACCA GGACAGCGGG TTGAATTCCT CGTGCAAGCG TGGAAGTCCA AGAAAACCCC 8220
   AATGGGGTTC TCGTATGATA CCCGCTGCTT TGACTCCACA GTCACTGAGA GCGACATCCG 8280
   TACGGAGGAG GCAATCTACC AATGTTGTGA CCTCGACCCC CAAGCCCGCG TGGCCATCAA 8340
   GTCCCTCACC GAGAGGCTTT ATGTTGGGGG CCCTCTTACC AATTCAAGGG GGGAGAACTG 8400
   CGGCTATCGC AGGTGCCGCG CGAGCGGCGT ACTGACAACT AGCTGTGGTA ACACCCTCAC 8460
   TTGCTACATC AAGGCCCGGG CAGCCTGTCG AGCCGCAGGG CTCCAGGACT GCACCATGCT 8520
   CGTGTGTGGC GACGACTTAG TCGTTATCTG TGAAAGCGCG GGGGTCCAGG AGGACGCGGC 8580
   GAGCCTGAGA GCCTTCACGG AGGCTATGAC CAGGTACTCC GCCCCCCCTG GGGACCCCCC 8640
   ACAACCAGAA TACGACTTGG AGCTCATAAC ATCATGCTCC TCCAACGTGT CAGTCGCCCA 8700
   CGACGGCGCT GGAAAGAGGG TCTACTACCT CACCCGTGAC CCTACAACCC CCCTCGCGAG 8760
   AGCTGCGTGG GAGACAGCAA GACACACTCC AGTCAATTCC TGGCTAGGCA ACATAATCAT 8820
   GTTTGCCCCC ACACTGTGGG CGAGGATGAT ACTGATGACC CATTTCTTTA GCGTCCTTAT 8880
   AGCCAGGGAC CAGCTTGAAC AGGCCCTCGA TTGCGAGATC TACGGGGCCT GCTACTCCAT 8940
   AGAACCACTT GATCTACCTC CAATCATTCA AAGACTCCAT GGCCTCAGCG CATTTTCACT 9000
   CCACAGTTAC TCTCCAGGTG AAATTAATAG GGTGGCCGCA TGCCTCAGAA AACTTGGGGT 9060
   ACCGCCCTTG CGAGCTTGGA GACACCGGGC CCGGAGCGTC CGCGCTAGGC TTCTGGCCAG 9120
   AGGAGGCAGG GCTGCCATAT GTGGCAAGTA CCTCTTCAAC TGGGCAGTAA GAACAAAGCT 9180
   CAAAC 9185

### (2) INFORMATION FOR SEQ ID NO:4:

(i) SEQUENCE CHARACTERISTICS:
   (A) LENGTH: 480 base pairs
   (B) TYPE: nucleic acid
   (C) STRANDEDNESS: double
   (D) TOPOLOGY: linear
(ii) MOLECULE TYPE: cDNA
(vi) ORIGINAL SOURCE:
   (A) ORGANISM: Hepatitis C Virus
   (B) STRAIN: HCV1
(vii) IMMEDIATE SOURCE:
   (B) CLONE: DNA 35
(ix) FEATURE:
   (A) NAME/KEY: CDS
   (B) LOCATION: 1..480
(xi) SEQUENCE DESCRIPTION: SEQ ID NO:4:
   TCC ATT GAG ACA ATC ACG CTC CCC CAG GAT GCT GTC TCC CGC ACT CAA      48
   Ser Ile Glu Thr Ile Thr Leu Pro Gln Asp Ala Val Ser Arg Thr Gln
     1               5                  10                  15
   CGT CGG GGC AGG ACT GGC AGG GGG AAG CCA GGC ATC TAC AGA TTT GTG      96
   Arg Arg Gly Arg Thr Gly Arg Gly Lys Pro Gly Ile Tyr Arg Phe Val
                20                  25                  30
   GCA CCG GGG GAG CGC CCC TCC GGC ATG TTC GAC TCG TCC GTC CTC TGT     144
   Ala Pro Gly Glu Arg Pro Ser Gly Met Phe Asp Ser Ser Val Leu Cys
            35                  40                  45
   GAG TGC TAT GAC GCA GGC TGT GCT TGG TAT GAG CTC ACG CCC GCC GAG     192
   Glu Cys Tyr Asp Ala Gly Cys Ala Trp Tyr Glu Leu Thr Pro Ala Glu
        50                  55                  60
   ACT ACA GTT AGG CTA CGA GCG TAC ATG AAC ACC CCG GGG CTT CCC GTG     240
   Thr Thr Val Arg Leu Arg Ala Tyr Met Asn Thr Pro Gly Leu Pro Val
    65                  70                  75                  80
   TGC CAG GAC CAT CTT GAA TTT TGG GAG GGC GTC TTT ACA GGC CTC ACT     288
   Cys Gln Asp His Leu Glu Phe Trp Glu Gly Val Phe Thr Gly Leu Thr
                    85                  90                  95
   CAT ATA GAT GCC CAC TTT CTA TCC CAG ACA AAG CAG AGT GGG GAG AAC     336
   His Ile Asp Ala His Phe Leu Ser Gln Thr Lys Gln Ser Gly Glu Asn
               100                 105                 110
   CTT CCT TAC CTG GTA GCG TAC CAA GCC ACC GTG TGC GCT AGG GCT CAA     384
   Leu Pro Tyr Leu Val Ala Tyr Gln Ala Thr Val Cys Ala Arg Ala Gln
           115                 120                 125
   GCC CCT CCC CCA TCG TGG GAC CAG ATG TGG AAG TGT TTG ATT CGC CTC     432
   Ala Pro Pro Pro Ser Trp Asp Gln Met Trp Lys Cys Leu Ile Arg Leu
       130                 135                 140
   AAG CCC ACC CTC CAT GGG CCA ACA CCC CTG CTA TAC AGA CTG GGC GCT     480
   Lys Pro Thr Leu His Gly Pro Thr Pro Leu Leu Tyr Arg Leu Gly Ala
   145                 150                 155                 160

### (2) INFORMATION FOR SEQ ID NO:5:

(i) SEQUENCE CHARACTERISTICS:
   (A) LENGTH: 160 amino acids
   (B) TYPE: amino acid
   (D) TOPOLOGY: linear
(ii) MOLECULE TYPE: protein
(xi) SEQUENCE DESCRIPTION: SEQ ID NO: 5:
   Ser Ile Glu Thr Ile Thr Leu Pro Gln Asp Ala Val Ser Arg Thr Gln
    1               5                  10                  15
   Arg Arg Gly Arg Thr Gly Arg Gly Lys Pro Gly Ile Tyr Arg Phe Val
               20                  25                  30
   Ala Pro Gly Glu Arg Pro Ser Gly Met Phe Asp Ser Ser Val Leu Cys
           35                  40                  45
   Glu Cys Tyr Asp Ala Gly Cys Ala Trp Tyr Glu Leu Thr Pro Ala Glu
       50                  55                  60
   Thr Thr Val Arg Leu Arg Ala Tyr Met Asn Thr Pro Gly Leu Pro Val
   65                  70                  75                  80
   Cys Gln Asp His Leu Glu Phe Trp Glu Gly Val Phe Thr Gly Leu Thr
                   85                  90                  95
   His Ile Asp Ala His Phe Leu Ser Gln Thr Lys Gln Ser Gly Glu Asn
               100                 105                 110
   Leu Pro Tyr Leu Val Ala Tyr Gln Ala Thr Val Cys Ala Arg Ala Gln
           115                 120                 125
   Ala Pro Pro Pro Ser Trp Asp Gln Met Trp Lys Cys Leu Ile Arg Leu
       130                 135                 140
   Lys Pro Thr Leu His Gly Pro Thr Pro Leu Leu Tyr Arg Leu Gly Ala
   145                 150                 155                 160

## Description

### Technical Field

The invention relates to *in vitro* cell systems for the replication of a virus; more specifically, it relates to cell lines in which an etiologic agent of non-A non-B hepatitis (NANBH), hepatitis C virus (HCV) will replicate.

### References Cited in the Application

ATCC CATALOG OF CELL LINES & HYBRIDOMAS, 6th edition, 1988 (American Type Culture Collection, Rockville, Md.).
BASIC & CLINICAL IMMUNOLOGY, 6th edition (Stites, Stobo, and Wells, eds., Appleton and Lange, Norwalk Conn/Los Altos, Ca, 1987).
Boggs and Winkelstein, WHITE CELL MANUAL, edition 4 (F.A. Davis Co., Phila., 1983.
Choo et al. (1989), Science 244:359 Diggs, Sturm, and Bell (1985), THE MORPHOLOGY OF HUMAN BLOOD CELLS (Abbott Laboratories, Abbott Park, I1.)
DNA CLONING, VOLUMES I AND II (D.N Glover ed. 1985).
Freshney (1987), CULTURE OF ANIMAL CELLS: A MANUAL OF BASIC TECHNIQUE (Alan R. Liss, Inc., N.Y.).
GENE TRANSFER VECTORS FOR MAMMALIAN CELLS (J.H. Miller and M.P. Calos eds. 1987, Cold Spring Harbor Laboratory).
Houghton et al., EP-A-318 216 and EP-A-388 232.
HANDBOOK OF EXPERIMENTAL IMMUNOLOGY, VOLUMES I-IV (D.M. Weir and C. C. Blackwell eds 1986).
Hellings et al., J. Virol. Meth. (1985), 10:321-326.
Hunt (1987), in LYMPHOCYTES: A PRACTICAL APPROACH (Klaus, ed., IRL Press Ltd., Washington D.C.), pp. 1-32.
IMMOBILIZED CELLS AND ENZYMES (IRL Press, 1986).
Kuo et al. (1989), Science 244:362.
Laemmli (1970), Nature 227:680.
LYMPHOCYTES: A PRACTICAL APPROACH (Klaus, ed., 1987, IRL Press, Ltd., Washington, D.C.)
LYMPHOKINES AND INTERFERONS: A PRACTICAL APPROACH (Clemens, Morris, and Gearing, eds., 1987, IRL Press, Ltd. Washington D.C.).
Maniatis, Fitsch & Sambrook, MOLECULAR CLONING; A LABORATORY MANUAL (1982).
Mayer and Walker, eds. (1987), IMMUNOCHEMICAL METHODS IN CELL AND MOLECULAR BIOLOGY (Academic Press, London).
McKeehan et al. (1978), Biochem. Biophys. Res. Comm. 80:1013.
METHODS IN ENZYMOLOGY (Academic Press, Inc.); also, Methods in Enzymology Vol. 154 and Vol. 155 (Wu and Grossman, and Wu, eds., respectively)
NUCLEIC ACID HYBRIDIZATION (B.D. Hames & S.J. Higgins eds. 1984).
OLIGONUCLEOTIDE SYNTHESIS (M.J. Gait ed, 1984). Perbal, A PRACTICAL GUIDE TO MOLECULAR CLONING (1984).
Saiki et al. (1986), Nature 324:163.
Seto et al., National Technical Information Service Report No. PAT-APPL-7-234 641 (198_).
Scopes, (1987), PROTEIN PURIFICATION: PRINCIPLES AND PRACTICE, Second Edition (Springer-Verlag, N.Y.).
VIROLOGY: A PRACTICAL APPROACH (Mahy, ed., 1985, IRL Press, Ltd., Washington, D.C.)

### Background

Non-A, Non-B hepatitis (NANBH) is a transmissible disease or family of diseases that are believed to be viral-induced, and that are distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents. A viral agent, HCV, has been identified as an etiologic agent of NANBH.

The demand for sensitive, specific methods for screening and identifying carriers of NANBV and NANBV contaminated blood or blood products is significant. Post-transfusion hepatitis (PTH) occurs in approximately 10% of transfused patients, and NANBH accounts for up to 90% of these cases. The major problem in this disease is the frequent progression to chronic liver damage (25-55%).

Patient care as well as the prevention of transmission of NANBH by blood and blood products or by close personal contact require reliable diagnostic and prognostic tools to detect nucleic acids, antigens and antibodies related to NANBV. In addition, there is also a need for effective vaccines and immunotherapeutic therapeutic agents for the prevention and/or treatment of the disease. EP-A-318 216 provides a family of cDNA sequences derived from Hepatitis C virus.

Tissue culture cells infected with an etiologic agent which causes NANBH would provide a source of viral polypeptides which could be used for immunoassays and/or for vaccines. In addition, they could be useful in drug screening programs in the development of antiviral agents for treating the disease.

Immortalized human or non-human primate cell lines which are derived from liver, and are purportedly infected with an etiologic agent causitive of NANBH have been reported in WO/87/05930, filed 1 April 1987, and published 8 October 1987. These cell lines are reportedly non-lymphocytic in nature. In addition, it has not been established that the agent which replicates in these cells is causitive of the disease.

It has also been reported that a preparation of mononuclear leukocytes, which purportedly were mainly lymphocytes, isolated from a patient with NANBH, caused NANB when infused into a susceptible chimpanzee. Hellings et al. (1985). However, proof of NANBH infection was indirect, in that it rested primarily on morphological and histological changes in the purportedly infected chimpanzee. Moreover, only one of the two chimpanzees receiving the blood cells was purportedly infected.

### Disclosure of the Invention

The present invention is directed to cells that may be used to propagate HCV in culture. The present invention is also directed to viral antigens produced by such cultures, and use such antigens, for example, in diagnostics.

Accordingly, one aspect of the invention is a cell line infected with HCV, wherein the cells in the cell line are B lymphocytes or T lymphocytes and are capable of replicating HCV.

Another aspect of the invention is a method of preparing a composition for the detection of an anti-HCV antibody which comprises: purifying or partially purifying HCV virus particles or polypeptides from a cell line of the invention; and fixing said particles or polypeptides to a solid phase.

The composition prepared by the invention may be used in an immunoassay for detecting antibodies directed against an HCV antigen comprising:
(a) incubating a sample suspected of containing anti-HCV antibodies with an HCV antigen produced by an *in vitro* cell line, under conditions which allow the formation of an antibody-antigen complex; and
(b) detecting the antibody-antigen complex containing the HCV antigen.

Another aspect of the invention is a method for screening for an agent which inhibits HCV replication comprising:
(a) providing a composition comprised of a putative anti-viral agent;
(b) providing a cell line capable of replicating HCV;
(c) providing a composition comprised of infective HCV;
(d) incubating the compositions of (a) and (c) with the cell line of (b) under conditions which would, in the absence of (a) allow infection of and replication of HCV in the cell line; and
(e) detecting an inhibition of viral replication after the incubating in (d), wherein the cells in the cell line of (b) are B lymphocytes or T lymphocytes.

Still another application of the invention is a method for proliferating virus comprising growing in culture HCV infected cells.

### Brief Description of the Drawings

Figure 1 shows a composite sequence of the HCV cDNA sense strand derived from HCV strain HCV1.

Figure 2 shows the nucleotide sequence of HCV cDNA clone 35, and the amino acids encoded therein.

### Modes for Carrying Out the Invention

### I. Definitions

The term "hepatitis C virus" has been reserved by workers in the field for an heretofore unknown etiologic agent of NANBH. Accordingly, as used herein, "hepatitis C virus" (HCV) refers to an agent causative of NANBH, which was formerly referred to as NANBV and/or BB-NANBV. The terms HCV, NANBV, and BB-NANBV are used interchangeably herein. As an extension of this terminology, the disease caused by HCV, formerly called NANB hepatitis (NANBH), is called hepatitis C. The terms NANBH and hepatitis C may be used interchangeably herein.

The term "HCV", as used herein, denotes a viral species of which pathogenic strains cause NANBH, and attenuated strains or defective interfering particles derived therefrom. The HCV genome is comprised of RNA. It is known that RNA containing viruses have relatively high rates of spontaneous mutation, i.e., reportedly on the order of 10⁻³ to 10⁻⁴ per incorporated nucleotide (Fields & Knipe (1986)). Therefore, there can be multiple strains, which may be virulent or avirulent, within the HCV species. It is believed that HCV is a Flavi-like virus. The morphology and composition of Flavivirus particles are known, and are discussed in Brinton (1986). Generally, with respect to morphology, Flaviviruses contain a central nucleocapsid surrounded by a lipid bilayer. Virions are spherical and have a diameter of about 40-50 nm. Their cores are about 25-30 nm in diameter. Along the outer surface of the virion envelope are projections that are about 5-10 nm long with terminal knobs about 2 nm in diameter.

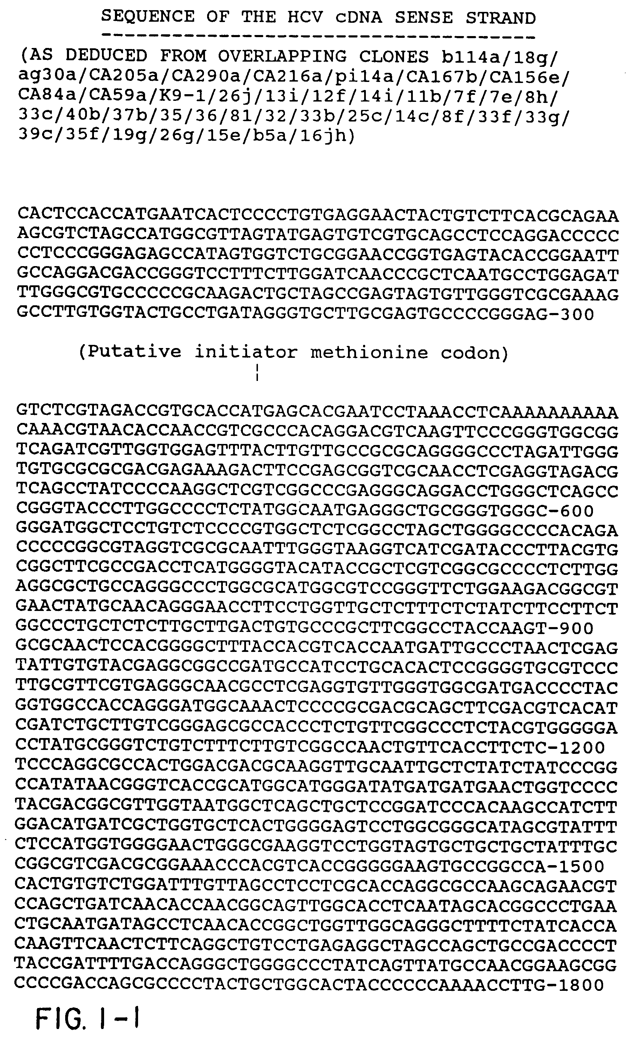

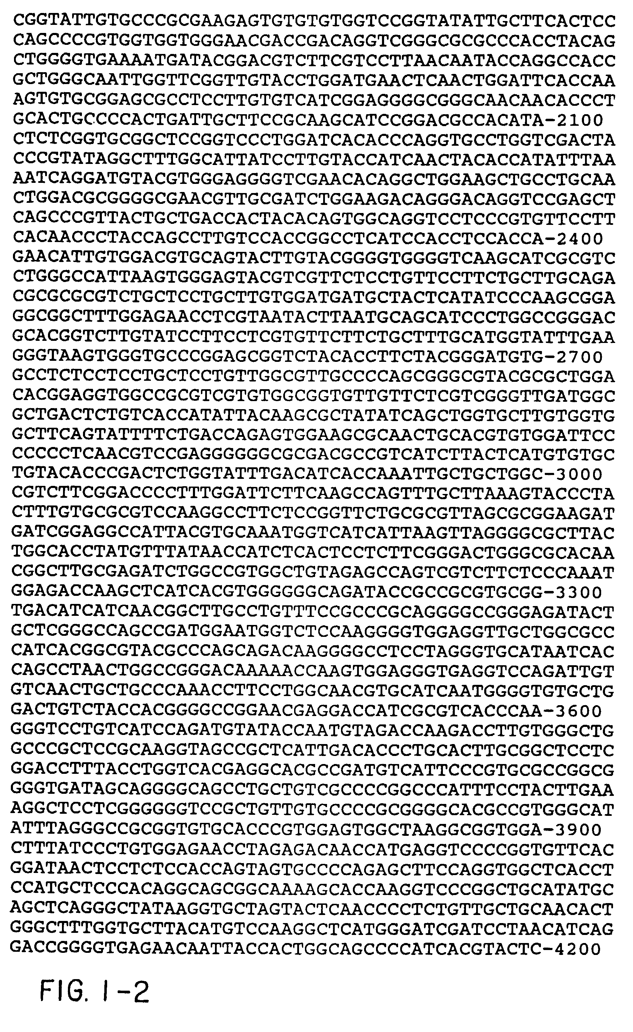

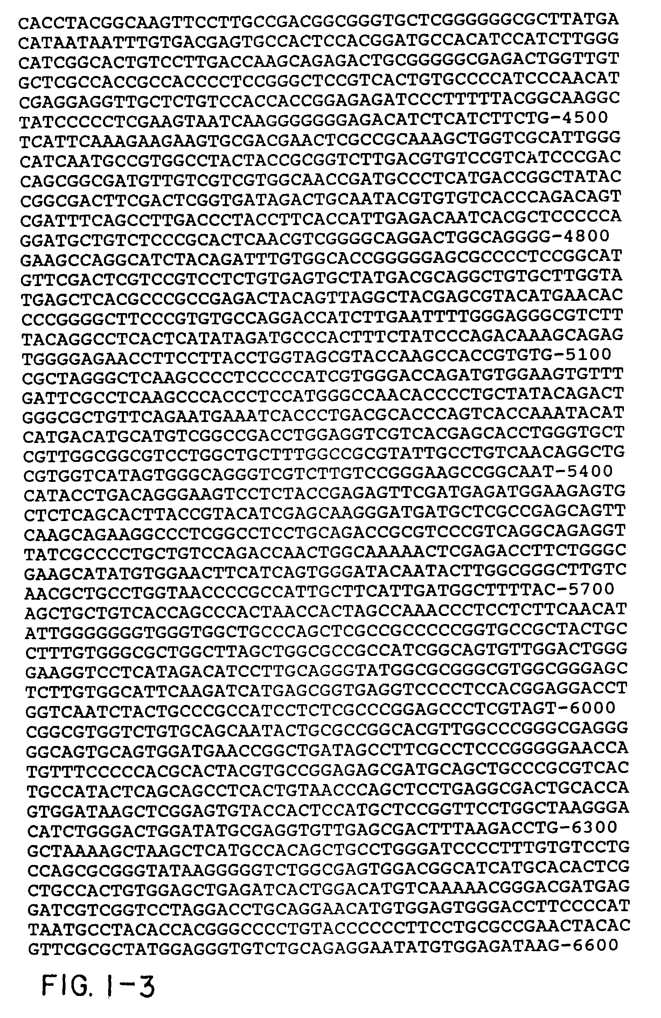

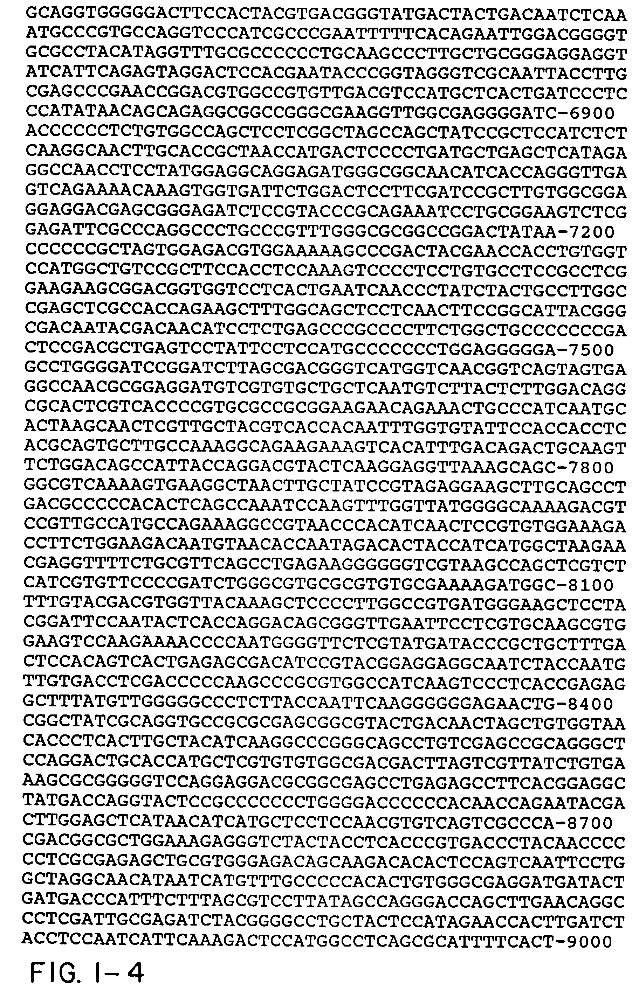

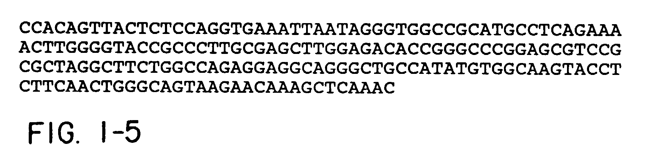

cDNAs to the genome of a strain of HCV, denoted HCV1, are described in EP-A-388 232. See also EPO Publication No. 0 318 216 and PCT App. No. US88/04125. A composite sequence of the HCV cDNA sense strand is shown in Figure 1. Different strains or isolates of HCV are expected to contain variations at the amino acid and nucleic acids levels compared with HCV1. Many isolates are expected to show much (i.e., more than about 40%) homology in the total amino acid sequence compared with HCV1. However, it may also be found that other less homologous HCV isolates would be defined as HCV strains according to various criteria such as an ORF encoding a polyprotein similar in size to that of HCV1, an encoded polyprotein of similar hydrophobic and antigenic character to that of HCV1, and the presence of co-linear peptide sequences that are conserved with HCV1.

HCV encodes at least one epitope which is immunologically identifiable with an epitope encoded in the HCV1 genome. The epitope is unique to HCV when compared to other known Flaviviruses. The uniqueness of the epitope may be determined by its immunological reactivity with anti-HCV antibodies and lack of immunological reactivity with antibodies to other Flavivirus species. Methods for determining immunological reactivity are known in the art, for example, by radioimmunoassay, by ELISA assay, and by hemagglutination. Examples of suitable techniques for determining immunological reactivity of HCV epitopes are described in EP-A-388 232, in Choo et al. (1989), and in Kuo et al. (1989).

In addition to the above, the following parameters of nucleic acid homology and amino acid homology are applicable, either alone or in combination, in identifying a strain or isolate as HCV. Since HCV strains and isolates are evolutionarily related, it is expected that the overall homology of the genomes at the nucleotide level probably will be about 40% or greater, probably about 60% or greater, and even more probably about 80% or greater; and in addition that there will be corresponding contiguous sequences of at least about 13 nucleotides. The correspondence between the putative HCV strain genomic sequence and the HCV1 cDNA sequence can be determined by techniques known in the art. For example, they can be determined by a direct comparison of the sequence information of the polynucleotide from the putative HCV, and the HCV cDNA sequence(s) described herein. For example, also, they can be determined by hybridization of the polynucleotides under conditions which form stable duplexes between homologous regions (for example, those which would be used prior to S₁ digestion), followed by digestion with single stranded specific nuclease(s), followed by size determination of the digested fragments.

Because of the evolutionary relationship of the strains or isolates of HCV, putative HCV strains or isolates are identifiable by their homology at the polypeptide level. Generally, HCV strains or isolates are expected to be more than about 40% homologous, probably more than about 70% homologous, and even more probably more than about 80% homologous, and some may even be more than about 90% homologous at the polypeptide level. The techniques for determining amino acid sequence homology are known in the art. For example, the amino acid sequence may be determined directly and compared to the sequences provided herein. Alternatively the nucleotide sequence of the genomic material of the putative HCV may be determined (usually via a cDNA intermediate), the amino acid sequence encoded therein can be determined, and the corresponding regions compared.

As used herein, a polynucleotide "derived from" a designated polynucleotide sequence refers to a polynucleotide sequence which is comprised of a sequence of approximately at least about 6 nucleotides, preferably at least about 8 nucleotides, more preferably at least about 10-12 nucleotides, and even more preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence which is unique to an HCV genome. Whether or not a sequence is unique to the HCV genome can be determined by techniques known to those of skill in the art. For example, the sequence can be compared to sequences in databanks, e.g., Genebank, to determine whether it is present in the uninfected host or other organisms. The sequence can also be compared to the known sequences of other viral agents, including those which are known to induce hepatitis, e.g., HAV, HBV, and HDV, and to other members of the Flaviviridae. The correspondence or non-correspondence of the derived sequence to other sequences can also be determined by hybridization under the appropriate stringency conditions. Hybridization techniques for determining the complementarity of nucleic acid sequences are known in the art, and are discussed *infra.* See also, for example, Maniatis et al. (1982). In addition, mismatches of duplex polynucleotides formed by hybridization can be determined by known techniques, including for example, digestion with a nuclease such as S1 that specifically digests single-stranded areas in duplex polynucleotides. Regions from which typical DNA sequences may be "derived" include but are not limited to, for example, regions encoding specific epitopes, as well as non-transcribed and/or non-translated regions.

The derived polynucleotide is not necessarily physically derived from the nucleotide sequence shown, but may be generated in any manner, including for example, chemical synthesis or DNA replication or reverse transcription or transcription. In addition, combinations of regions corresponding to that of the designated sequence may be modified in ways known in the art to be consistent with an intended use.

The term "polynucleotide" as used herein refers to a polymeric form of nucleotides of any length, either ribonucleotides or deoxyribonucleotides. This term refers only to the primary structure of the molecule. Thus, this term includes double- and single-stranded DNA, as well as double- and single stranded RNA. It also includes modified, for example, methylation capping and/or by other modifications known in the art, including the substitution of an analog for one or more of the naturally occurring nucleotide bases, as well as unmodified forms of the polynucleotide.

The term "purified viral polynucleotide" refers to a viral genome or fragment thereof which is essentially free, i.e., contains less than about 50%, preferably less than about 70%, and even more preferably less than about 90% of polypeptides with which the viral polynucleotide is naturally associated. Techniques for purifying viral polynucleotides from viral particles are known in the art, and include for example, disruption of the particle with a chaotropic agent, differential extraction and separation of the polynucleotide(s) and polypeptides by ion-exchange chromatography, affinity chromatography, and sedimentation according to density.

An "open reading frame" (ORF) is a region of a polynucleotide sequence which encodes a polypeptide; this region may represent a portion of a coding sequence or a total coding sequence.

A "coding sequence" is a polynucleotide sequence which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, and recombinant polynucleotide sequences.

"Immunologically identifiable with/as" refers to the presence of epitope(s) and polypeptides(s) which are also present in the designated polypeptide(s), usually HCV proteins. Immunological identity may be determined by antibody binding and/or competition in binding; these techniques are known to those of average skill in the art, and are also illustrated *infra*.

As used herein, "epitope" refers to an antigenic determinant of a polypeptide; an epitope could comprise 3 or more amino acids in a spatial conformation which is unique to the epitope. Generally an epitope consists of at least 5 such amino acids, and more usually, consists of at least 8-10 such amino acids. Methods of determining the spatial conformation of amino acids are known in the art, and include, for example, X-ray crystallography and 2-dimensional nuclear magnetic resonance.

A polypeptide is "immunologically reactive" with an antibody when it binds to an antibody due to antibody recognition of a specific epitope contained within the polypeptide. Immunological reactivity may be determined by antibody binding, more particularly by the kinetics of antibody binding, and/or by competition in binding using as competitor(s) a known polypeptide(s) containing an epitope against which the antibody is directed. The techniques for determining whether a polypeptide is immunologically reactive with an antibody are known in the art.

As used herein, the term "immunogenic polypeptide" refers to a polypeptide that elicits a cellular and or humoral response, whether alone or when linked to a carrier in the presence or absence of an adjuvant.

The term "polypeptide" refers to a polymer of amino acids and does not refer to a specific length of the product; thus, peptides, oligopeptides, and proteins are included within the definition of polypeptide. This term also does not refer to or exclude post-expression modifications of the polypeptide, for example, glycosylations, acetylations, phosphorylations and the like.

An "individual", as used herein, refers to vertebrates, particularly members of the mammalian species, and includes but is not limited to domestic animals, sports animals, and primates, including humans.

As used herein, the "sense strand" or "plus strand" of a nucleic acid contains the sequence that has sequence homology to that of mRNA. The "antisense strand" or "minus strand" contains a sequence which is complementary to that of the "sense strand" or "plus strand".

As used herein, a "positive stranded genome" of a virus is one in which the genome, whether RNA or DNA, is single-stranded and which encodes a viral polypeptide(s). Examples of positive stranded RNA viruses include Togaviridae, Coronaviridae, Retroviridae, Picornaviridae, and Caliciviridae. Included also, are the Flaviviridae, which were formerly classified as Togaviradae. See Fields & Knipe (1986).

As used herein, a "replicative intermediate" of an HCV genome refers to an RNA strand or fragment thereof, which is complementary to the viral genome, and which is synthesized during the course of viral replication; the replicative intermediate functions as a template for the synthesis of positive RNA strands.

As used herein, "purified HCV" refers to a preparation of HCV which has been isolated from the cellular constituents with which the virus is normally associated, and from other types of viruses which may be present in the infected tissue. The techniques for isolating viruses are known to those of skill in the art, and include, for example, centrifugation and affinity chromatography; a method of preparing purified HCV is discussed *infra.*

As used herein, a "biological sample" refers to a sample of tissue or fluid isolated from an individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of *in vitro* cell culture constituents (including but not limited to conditioned medium resulting from the growth of cells in cell culture medium, putatively virally infected cells, recombinant cells, and cell components).

The term "oligonucleotide", as used herein, refers to primers, probes, oligomer fragments to be detected, oligomer controls and unlabeled blocking oligomers, and is defined as a molecule comprised of three or more deoxyribonucleotides or ribonucleotides, typically contains more than four, typically a maximum of about 100 nucleotides. Its exact size will depend upon many factors, which in turn depend on the ultimate function or use of the oligonucleotide.

The term "primer", as used herein, refers to an oligonucleotide which is capable of acting as a point of initiation of polynucleotide synthesis along a complementary strand when placed under conditions in which synthesis of a primer extension product which is complementary to a polynucleotide is catalyzed. Such conditions include the presence of four different nucleotide triphosphates or nucleoside analogs and an agent for polymerization such as DNA polymerase or reverse transcriptase, in an appropriate buffer ("buffer" includes substituents which are cofactors, or which affect pH, ionic strength, etc.), and at a suitable temperature.

As used herein, the term "target region" refers to a region of the nucleic acid which is to be amplified and/or detected.

As used herein, the term "probe" refers to a polynucleotide or oligonucleotide which forms a hybrid structure with a sequence in a target region, due to complementarity of at least one sequence in the probe with a sequence in the target region. Preferably the probe, however, does not contain a sequence complementary to sequence(s) used to prime the polymerase chain reaction.

As used herein, the term "thermostable nucleotide polymerase" refers to an enzyme which is relatively stable to heat when compared to nucleotide polymerases from *E. coli* and which catalyzes the polymerization of nucleotides. Generally, the enzyme will initiate synthesis at the 3'-end of the target sequence utilizing the primer, and will proceed in the 5'-direction along the template until synthesis terminates. A representative thermostable enzyme isolated from *Thermus aquaticus* (Taq), and method for using it is described in Saiki et al. (1988).

As used herein, the term "viral RNA", which includes HCV RNA, refers to RNA from the viral genome, fragments thereof, transcripts thereof, and mutant sequences derived therefrom.

As used herein, "cell line" refers to a propagated culture of cells after the first subculture, thus there are various embodiments including but not limited to individual cells, harvested cells, and cultures containing cells so long as these are are derived from cells of the cell line referred to. By "derived" is meant progeny or issue. It is further known in the art that spontaneous or induced changes can occur in karyotype during storage or transfer. Therefore, cells derived from the cell line referred to may not be precisely identical to the ancestral cells or cultures, and the cell line referred to includes such variants. The term "cell lines" includes immortalized cells. Preferably, cell lines exclude cells which are derived by the hybridization of three cell types, for example, a trioma.

As used herein, an "*in vitro*" cell system refers to cells which have replicated *in vitro*, thus the term includes primary cultures and cell lines.

As used herein, the term "blood cell" refers to cells found either circulating in the blood, in tissue, or in bone marrow, and includes cells of the myelocytic series, the monocytic series, the megakaryocytic series, the erythrocytic series, the lymphocytic series, and the plasmocytic series. The morphology by which these cells can be distinguished is discussed in THE MORPHOLOGY OF HUMAN BLOOD CELLS (1985).

As used herein, the term "peripheral blood cell" refers to a cell found circulating in the blood, and refers generally to eosinophils, neutrophils (both band and segmented), basophils, monocytes, thrombocytes, erythrocytes, lymphocytes, and plasmocytes.

As used herein, the term "leukocyte" refers to to a heterogeneous group of blood cell types which includes everything except erythrocytes and platelets.

As used herein, the term "lymphocyte" refers to a spherical cell, 6-12 µm in diameter, involved in immune responses, with a large, round, and deeply staining nucleus and very little cytoplasm. Lymphocytes include B lymphocytes and T lymphocytes.

As used herein, "B lymphocytes" are vertebrate white blood cells which, when mature, are involved in the production of antibody. Mature virgin B lymphocytes (i.e., those that have not encountered antigen) are characterized by the presence of the following surface markers: monomeric IgM receptors for antigen, Fc receptors for IgG; and receptors for the activated form of C3b component of complement.

Precursor B lymphocytes lack immunoglobulin products but may express other characteristic molecules that can be identified by specific monoclonal antibodies. These cells divide and undergo rapid transition to become large lymphoblasts with cytoplasmic u heavy chains but no light chains. The pre-B cell phenotype (cytoplasmic u chain ⁺, light chain ⁻, surface Ig⁻) can first be demonstrated in lymphoblasts actively engaged in DNA synthesis. Human pre-B cells express HLA-DR molecules on their surface and may also express receptors for the C3b complement fragment. However, they lack Fc recpetors for IgG and have very few C3d/EBV receptors. Precursor B lymphocytes can also be recognized by their capacity to bind peanut agglutinin.

Mature B lymphocytes may be either activated or resting. Activated B cells express several types of molecules that are not detected on resting B cells. These marker molecules are known by those of skill in the art, and include, for example, interleukin-2 (IL-2) receptors, and transferrin receptors (the latter are expressed by all kinds of dividing cells). Activation of B cells provokes loss of membrane IgD, and both Fc_{γ} and C3b receptors are diminished on external membranes, whereas those for C3d/Epstein Barr Virus (EB)V are not significantly affected.

After antigen or mitogen stimulation, B lymphocytes may proceed along either of 2 branches of a differentiation pathway. They can differentiate into plasma cells, or they can divide and then return to a resting state as memory B cells. Memory B cells have had a loss of IgD during activation; this molecule is not resynthesized by cells entering the memory cell pool.

"Plasma cells" are terminally differentiated B lymphocytes. These cells are of ovoid shape, with an eccentric spoke-wheel nucleus, and intensely basophilic cytoplasm. When stained with fluorochrome-labeled antibodies to immunoglobulin determinants, fixed plasma cells display intense fluorescence throught their cytoplasm. Membrane-bound immunoglobulin and DR molecules are scant, and receptors for Fc_{γ}, C3b, C3d, or EBV are generally undetectable.

A "plasmablast" is both morphologically and functionally between the activated lymphocyte (also called B lymphoblast) and plasma cell stages. The cells are large, with a greater nuclear:cytoplasmic ratio than plasma cells. Membrane-bound immunoglobulins and Fc_{γ} receptors are present.

As used herein, a "T lymphocyte" is a thymus-derived cell which may participate in a variety of cell-mediated immune responses. There are a variety of types of T lymphocytes, which are distinguishable by their function, and by their cell surface markers. Methods for distinguishing the classes of T lymphocytes, which include those of the T "helper" and T "suppressor" types are known in the art. For example, monoclonal antibodies can be used to detect cell surface antigens, including the CD (cluster of differentiation) antigens. The T cell types marked by the CD antigens, and monoclonal antibodies which detect the specific antigens are shown in Table 1.

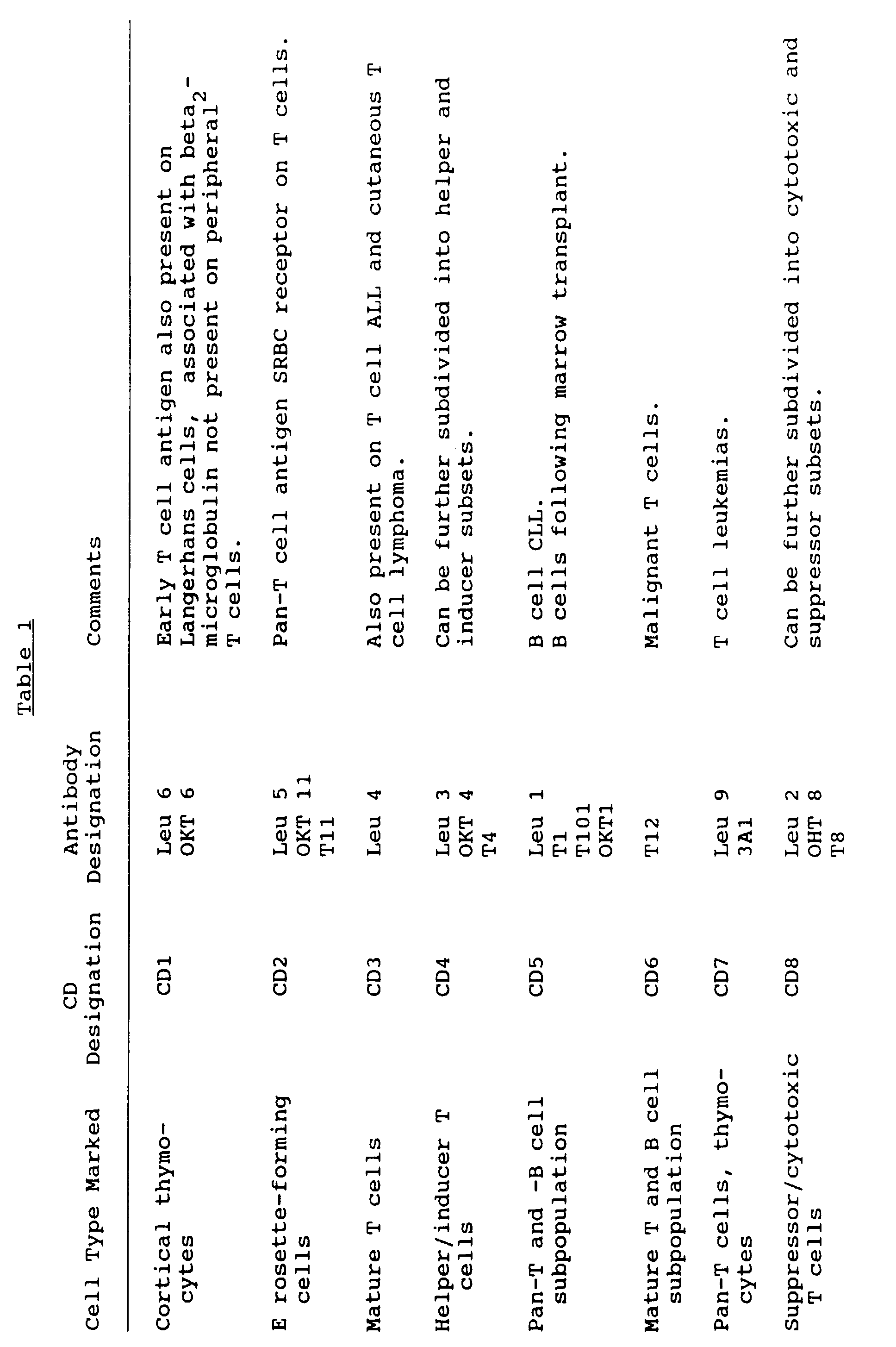

As used herein, "large granular lymphocyte" (LGL), also called natural killer (NK) cells, are another type of lymphocyte. These cells comprise a discrete population of large lymphocytes that can be distinguished morphologic features, for example, by characteristic azurophilic granules in their cytoplasm. In addition, LGL can be identified by methods which utilize specific monoclonal antibodies. Several monoclonal antibodies are available that detect either Fc receptors (Leu 11) or specific differentiation antigens (Leu 7, HNK-1, NKH-1, NKH-2) present on these cells. Some LGL cells also express antigens from the CD2 T cell family. Methods for functional testing are also known in the art, and are based on the ability of these nonimmune cells to kill special target cells, for example, erythroleukemia cell line K562. Methods of measuring cytotoxocity are known in the art, and include, for example the ⁵¹Cr release assay.

As used herein, "monocytes" denotes large agranulocytic leukocyte blood cells which are phagocytic. In humans, these cells are approximately 10-12 µm in diameter, with oval nuclei. Monocytes which are in tissue are known as macrophages. Monocytes/macrophages may be identified by their morphology. They are larger than granulocytes and most lymphocytes. They have round or kidney-shaped nuclei with fine, lightly stained granules. They may be detected by staining for their nonspecfic esterase, or alpha-nahthol esterase. Monoclonal antibodies directed at specific differentiation antigens are also available, and include, for example, Leu M3, and OKT M1. The term "monocytes" includes monoblasts and promonocytes. The cells of the mononuclear phagocyte system, and their localization, are shown in Table 2; methods for distinguishing the cell types are discussed in BASIC & CLINICAL IMMUNOLOGY, 6th edition.

**Table 2**

| Cells of the Mononuclear Phagocyte System* | |
|---|---|
| Cells | Localization |
| Stem cells (committed) | Bone marrow |
| Monoblasts | Bone marrow |
| Promonocytes | Bone marrow |
| Monocytes | Bone marrow |
| Macrophages | Tissues |

| Normal state, free | |
|---|---|
| Histocytes | Connective tissues |
| Alveolar macrophages | Lung |
| Pleural and peritoneal macrophages | Serous cavities |

| Normal state, fixed | |
|---|---|
| Kupffer cells | Liver |
| Osteoclasts | Bone |
| Microglial cells | Nervous System |
| Synovial type A cells | Joints |
| Fixed tissue macrophages | Spleen, lymph nodes, bonemarrow, and other tissues |

| Inflammation | |
|---|---|
| Exudate macrophages | Any tissue |
| Activated macrophages | Any tissue |
| Elicited macrophages | Any tissue |
| Epitheloid cells | Any tissue |
| Multinucleated giant cells (Langerhans types and foreign body type) | Any tissue |

| | |
|---|---|
| * Adapted from Van Furth R (editor): Mononuclear Phagocytes: Functional Aspects, Martinus Nijhoff, 1980. | |

As used herein, the term "granulocyte" refers to a polymorphonuclear leukocyte which contains many cytoplasmic granules and a multilobed nucleus. Granulocytes include neutrophils, eosinophils, and neutrophils. Neutrophils are amoeboid in shape, and phagocytic, and stain with both basic and acidic dyes. Eosinophils and basophils contain cytoplasmic granules which stain with acid dyes and with basic dyes, respectively.

"Bone marrow cells" include both primary bone marrow tissue and bone marrow cell lines from mammals, such as primates and humans. Methods of culturing primary bone marrow cells *in vitro* are known, as well as bone marrow cell lines such as IM-9, KG-1 and KG-la. See, e.g., ATCC CATALOG, *supra,* at 361. Primary bone marrow cells may include, in addition to leukocytes and lymphocytes described elsewhere herein, progenitor cells such as pleuripotent stem cells. See, e.g., Civin U.S. Pat. No. 4,714,680.

### II. Description of the Invention

The invention described herein allows the replication of HCV in cell lines wherein the cells are B lymphocytes and T lymphocytes. In a method of the invention, peripheral blood cells which are infected with replicating virions are detected. Preferably, the detection is accomplished by identifying replicative intermediates of the viral genome in preparations containing HCV infected cells. Alternatively, the virions may be detected using immunoassays to detect a population of HCV infected cells. Cell lines wherein the cells are B lymphocytes and T lymphocytes infected with HCV are prepared; these cell lines are usually derived from blood cells or their precursors, more usually are derived from mononuclear cells or their precursors. The preparation of the infected cell line may be accomplished by a variety of methods, including the following. One option is to cultivate susceptible cells with concentrated culture fluids harvested from short term primary cultures of cells which are replicating the virus. Another option is to directly infect susceptible cells with isolated HCV particles. Still another option is to grow susceptible cells in contact with HCV infected cells. The susceptible cells may be from an established cell line, or may be primary cultures. Alternatively, infected cells from an individual with HCV may be fused with established cells from a cell line to produce a permanent cell line. Still alternatively, HCV infected blood cells isolated from an individual may be immortalized. Virions produced by the HCV infected cell lines are useful in immunoassays for the detection of anti-HCV antibodies, and as a substrate for the production of vaccines for treatment of HCV.

The practice of the present invention will employ, unless otherwise indicated, conventional techniques of molecular biology, microbiology, hematology, cell and organ culture, recombinant DNA, and immunology, which are within the skill of the art. Such techniques are explained fully in the literature. See, e.g., ATCC CATALOG OF CELL LINES & HYBRIDOMAS, 6th edition, 1988 (American Type Culture Collection, Rockville, Md.); BASIC & CLINICAL IMMUNOLOGY, 6th edition (Stites, Stobo, and Wells, eds., Appleton and Lange, Norwalk Conn/Los Altos, Ca, 1987); Boggs and Winkelstein, WHITE CELL MANUAL, edition 4 (F.A. Davis Co., Phila., 1983); Diggs, Sturm, and Bell (1985), THE MORPHOLOGY OF HUMAN BLOOD CELLS (Abbott Laboratories, Abbott Park, Il.); Freshney (1987), CULTURE OF ANIMAL CELLS: A MANUAL OF BASIC TECHNIQUE (Alan R. Liss, Inc., N.Y.); Maniatis, Fitsch & Sambrook, MOLECULAR CLONING; A LABORATORY MANUAL (1982); DNA CLONING, VOLUMES I AND II (D.N Glover ed. 1985); LYMPHOKINES AND INTEFERONS: A PRACTICAL APPROACH (Clemens, Morris, and Gearing, eds., 1987); LYMPHOCYTES: A PRACTICAL APPROACH (Klaus, ed., 1987); VIROLOGY: A PRACTICAL APPROACH (Mahy, ed., 1985); OLIGONUCLEOTIDE SYNTHESIS (M.J. Gait ed, 1984); NUCLEIC ACID HYBRIDIZATION (B.D. Hames & S.J. Higgins eds. 1984); IMMOBILIZED CELLS AND ENZYMES (IRL Press, 1986); B. Perbal, A PRACTICAL GUIDE TO MOLECULAR CLONING (1984); the series, METHODS IN ENZYMOLOGY (Academic Press, Inc.); GENE TRANSFER VECTORS FOR MAMMALIAN CELLS (J.H. Miller and M.P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), IMMUNOCHEMICAL METHODS IN CELL AND MOLECULAR BIOLOGY (Academic Press, London), Scopes, (1987), PROTEIN PURIFICATION: PRINCIPLES AND PRACTICE, Second Edition (Springer-Verlag, N.Y.), and HANDBOOK OF EXPERIMENTAL IMMUNOLOGY, VOLUMES I-IV (D.M. Weir and C. C. Blackwell eds 1986).

### II.A. Detection and Isolation of HCV Infected Cells from an Individual

HCV infected cells in biological samples are screened for the presence of HCV polypeptides or HCV polynucleotides. Usually the biological samples are blood samples, from which the cells infected with HCV and/or replicating HCV are isolated and partially purified utilizing techniques known in the art, for example, by sedimentation through Ficoll-Paque (Pharmacia Corp.) solutions.

The screening technique used to detect viral material must be sufficiently sensitive to detect very limited amounts. For example, the presence of virions may be detected by immunoassay, using antibodies directed against a virion polypeptide(s). A suitable antibody is described in EP-A-388 232. Methods for immunoassays, as well as methods for signal enhancement are known in the art

The HCV genomic material, and replicative intermediates thereof, may also be detected by nucleic acid hybridization. Usually the system will involve amplification techniques in the hybridization assay. Such techniques are known in the art. For example, the Enzo Biochemical Corporation "Bio-Bridge" system uses terminal deoxynucleotide transferase to add unmodified 3'-poly-dT-tails to a DNA probe. The poly dT-tailed probe is hybridized to the target nucleotide sequence, and then to a biotin-modified poly-A. PCT application 84/03520 and EP-A-124221 describe a DNA hybridization assay in which: (1) analyte is annealed to a single-stranded DNA probe that is complementary to an enzyme-labeled oligonucleotide; and (2) the resulting tailed duplex is hybridized to an enzyme-labeled oligonucleotide. EP-A-204510 describes a DNA hybridization assay in which analyte DNA is contacted with a probe that has a tail, such as a poly-dT tail, an amplifier strand that has a sequence that hybridizes to the tail of the probe, such as a poly-A sequence, and which is capable of binding a plurality of labeled strands. A particularly desirable technique may first involve amplification of the target HCV sequences in sera until the amplified sequences are at a detectable level. This may be accomplished, for example, by the polymerase chain reactions (PCR) technique described by Saiki et al. (1986), by Mullis, U.S. Patent No. 4,683,195, and by Mullis et al. U.S. Patent No. 4,683,202. The amplified sequence(s) may then be detected using a hybridization assay. Hybridization assays, which should detect sequences at the level of 10⁶/ml, utilize nucleic acid multimers which bind to single-stranded analyte nucleic acid, and which also bind to a multiplicity of single-stranded labeled oligonucleotides. A suitable solution phase sandwich assay which may be used with labeled polynucleotide probes, and the methods for the preparation of probes is described in EP-A-225 807.

A preferred method for detecting HCV genomic sequences is described in the Examples. The method utilizes primers and probes based upon a family of cDNA replicas of portions of the HCV genome; a composite sequence of the cDNA plus strand derived from this family of cDNAs is shown in Figure 1. The first step in the method is the synthesis of a cDNA to either the HCV genome or its replicative intermediate, using reverse transcriptase. If replicative intermediates are to be detected, the primer is complementary to only the negative strand of HCV RNA (i.e, the strand which is the replicative intermediate). After synthesis of the HCV cDNA, and prior to amplification, the RNA in the sample is degraded by techniques known in the art, for example, by alkali denaturation, or by treatment with an RNA specific RNase.

It is anticipated that variant strains of HCV will be present in the infected cells of various individuals. Therefore the detection methods should be directed to constant epitopes or genomic sequences of HCV.

Utilizing the HCV/cPCR method to screen fractions of blood cells separated on Ficoll-Paque, replicative intermediates of the HCV genome were detected in a partially purified composition of cells derived from peripheral blood (See *infra* in the Examples). This composition is comprised of lymphocytes, monocytes/macrophages, platelets, and small amounts of other white blood cell types. The sedimentation through Ficoll-Paque fractionates these cells, which band to the interface, from erythrocytes and granulocytes, which are of greater density.

Further identification of the infected cell types may be accomplished by utilizing techniques known in the art, for example by immunoassays which are directed to distinguish between the various types of blood cells. Monoclonal antibodies which are directed to surface epitopes on various lymphocytes, and monocytes/ macrophages are known in the art. Thus, HCV infected cells may be isolated and identified by immunologic binding to specific cell surface antigens.

### II.B. Preparation of In vitro Infected Cell Systems

A variety of methods may be used to prepare B lymphocyte or T lymphocyte cell lines, infected with HCV. Candidate cells for infection and propagation *in vitro* can be from any of the above-mentioned cell types, such as lymphocytes or bone marrow cells. The discovery that replicative intermediates of the HCV genome are present in leukocytes in the peripheral blood of infected humans is indicative of the cell type which may support viral replication *in vitro,* i.e., in at least one of the cell types in the population of leukocytes which band at the interface of a leukocyte suspension sedimented through Ficoll-Paque, i.e., mononucleocytes. (This procedure generally removes normal erythrocytes and granulocytes from peripheral blood samples). Further fractionation procedures may be used to separate the different types of mononucleocytes to determine the presence of HCV replicative intermediates and/or HCV antigens associated with these cell types. Procedures for separating different types of monocytes, e.g., various types of lymphocytes, and macrophages, are known in the art. See, e.g., LYMPHOCYTES: A PRACTICAL APPROACH (Klaus, ed.). These procedures may use monoclonal antibodies directed towards specific cell types; the monoclonal antibodies may be affixed to solid substrates, e.g., plates with wells, or magnetic beads. Alternatively, sorting techniques, which rely upon cell sorter instruments may be used. The selection may be either positive or negative to enrich and/or select the desired cells in the population of mononucleocytes. Based upon fractionation studies which isolated lymphocytes and macrophages from the monocyte population, it is suggested that a preferred cell type for replication may be lymphocytes and/or macrophages/monocytes.

Since replication of HCV occurs within mononucleocytes *in vivo*, it is expected that infected cell lines may be derived from known established cell lines of these types, many of which are available from the American Type Culture Collection, and which may include, for example, B cell lines (e.g., EBV transformed B cell derived lines such as B95-8, DAKIKI, LTR228, Mo-B, SC-1, WIL2-NS; B cell lines such as Ia2, BL-3, UC 729-6, Namalwa, U-937, Ramos (RA-1), BCL₁ clone 5B₁b, MC/CAR, IA2, YB2/0, CA3-F4, SHM-D33, CW13.20-3B3, BC9-E%, WM-115, WM 266-4, WEHI-231, WEHI-279, NFS-1.0 C-1); monocyte lines (e.g., THP-1, WEHI-274.1), mouse lymphoma lines (e.g., 2pk-3), pro-B lymphoblasts (e.g., NFS-70 C-10), pre-B lymphoblasts (e.g., NFS-25 C-3), T-cell derived lines (e.g., CTLL-2, HuT 102, D10.G4.1, LBRM-33 clone 4A2, HuT78, Cl.Ly1⁺2⁻/9, UCD-MLA-144, 6T-CEM 20, 6T-CEM, CCRF-CEM, H9/HTLV-IIIB, CCRF-SB; CCRF-HSB-2, CEM-CM3, Mo, MOLT-3, MOLT-4, BW5147.3, BW5147.G.1.4, C1498, RAW8.1, CTLL-2, and BW5147.G.1.4.OUA^{R}.1). Additional human leukemic cell lines of interest include K562 and ARH-77. Conditions for the *in vitro* culture of these cells is described in ATCC CATALOG OF CELL LINES AND HYBRIDOMAS, 6th Edition, 1988.

Still another type of cell system in which HCV may be replicated is in immortal cell lines derived from primary cultures of mononucleocytes. Methods to obtain immortalized cell lines of types of mononucleocytes are known in the art. For example, methods for growing and immortalizing B cells and T cells in culture are described in LYMPHOCYTES: A PRACTICAL APPROACH (Klaus, ed.); LYMPHOKINES AND INTERFERONS (Clemens, Morris, and Gearing, eds), and Freshney (1987). For example, B-cell lines are often immortalized by infection with Epstein-Barr virus (EBV). This may be accomplished by incubating mononucleocytes from infected cells with the supernatant from an EBV infected cell line which is a chronic shedder of EBV, for example, ATCC CRL 1612 cells (B95-8).

The growth of B cells and T cells in culture often requires the presence of stimulatory protein factors (also called cytokines) in the culture medium; these factors are usually produced by a cell of another type. A supply of the cytokines may be provided in conditioned medium, i.e., cell culture supernatant from cells which produce the desired factors; or it may also be provided by feeder cells. The requirement may also be met by the provision of the purified cytokines. Cytokines include, for example, the following. Interleukin 1-alpha and beta (also called lymphocyte activating factor, epidermal cell derived thymocyte activating factor, and haemopoietin H1); these lymphokines are derived from multiple cell types, including monocytes, lymphocytes, and keratinocytes. Interleukin 1-alpha and beta have multiple actions, including stimulating interleukin 2 production and haematopoietic activity. Interleukin 2 (also called T-cell growth factor), derived from T-lymphocytes, and which stimulates T and B cell proliferation and differentiation, macrophage activation, and natural killer cell activation. Interleukin 3 (also called Multi-CSF), which is derived from T-lymphocytes, and which has a pluripotent effect on growth and differentiation, and which stimulates mast cell growth. Interleukin 4 (also called B-cell growth factor I), which is derived from T-lymphocytes, and which stimulates T and B cell proliferation and differentiation. Interleukin 5 (also called B-cell growth factor II), which is derived from T-lymphocytes, and which stimulates eosinophil differentiation (and which, in the mouse system, acts as a B-cell growth factor). Low molecular weight B-cell growth factor, which is derived from T-lymphocytes, and which stimulates B-cell proliferation. B-cell stimulatory factor 2 (also called interferon beta-2), which is derived from T-lymphocytes, and which stimulates B cell differentiation. Interferon-γ (also called immune interferon), which is produced by T-lymphocytes, and which activates macrophages.

Infection of a susceptible cell line with HCV may be accomplished by any one of several techniques which are used in the art to infect cells with virus, particularly with an RNA virus, and more particularly with a Flavivirus. Generally, virus particles adsorb to cultured cells at physiological temperatures. The virus penetrates, uncoats, replicates its nucleic acid in the nucleus or cytoplasm, and after transcription and translation assembles progeny virions which egress either by cell lysis or budding. The number of progeny virus particles released from one cell is referred to as the burst size. Virus is titrated by adsorbing serial tenfold dilutions to cells and counting the resultant plaques after appropriate incubation at the permissive temperature.

Viral infection of susceptible cells may be accomplished by cocultivation of the susceptible cells and HCV infected cells. The HCV infected cells may be from a primary culture. Usually the HCV infected cells and the primary cells are incubated together in approximately equal numbers in an appropriate cell culture medium. The medium may contain Polybrene, or a functionally similar substance, in an appropriate concentration. The incubation is of sufficient time and at the appropriate temperature to allow the newly replicated virus from the HCV infected cells to allow attachment and uptake by the susceptible cells.

The infection of susceptible cells may also be accomplished using virus containing cell-free supernatants of HCV infected cells. Generally the susceptible cells are pretreated with an appropriate concentration of Polybrene (or a functionally similar substance), and then exposed to concentrated culture fluids harvested from short-term primary cultures of HCV infected cells derived from infected individuals.

Susceptible cells may also be infected with virus isolated from HCV containing biological samples. Suitable methods for the isolation of viruses, particularly RNA containing viruses, and more particularly Togaviruses and Flaviviruses are known in the art, for example, see VIROLOGY: A PRACTICAL APPROACH (Mahy, ed.). Also described therein are cells which may be suitable for infection, and growth conditions for these cells.

Another method for preparing HCV infected cells *in vitro* may be by the fusion of HCV infected mononuclear cells with susceptible cells. Methods for fusing somatic cells are known in the art, as are methods for the selection of fused cells. For example, fusion may be catalyzed by the presence of virus, e.g., EBV, Sendai virus, and the like, or by the presence of chemicals, e.g., polyethylene glycol, which induce fusion. The susceptible cell may carry a marker which prevents its growth in the selection media, e.g., the cells may be phenotypically negative for thymidine kinase. Selection is then in media which requires thymidine kinase, e.g., HAT medium. This method is particularly suitable if the HCV infected mononuclear cells isolated from the infected individual do not replicate in *vitro.* A suitable method for the preparation fused cells which may replicate HCV is that described in WO 87/05930, except that the tissue specific cell (reportedly a hepatocyte) which is used to form the trioma is replaced with an HCV infected mononucleocyte.

Yet another method of preparing an HCV infected cell line is by immortalization of infected cells isolated from an HCV infected individual. Generally, mononuclear cells are isolated from peripheral blood. If desired, the mononuclear cell population may be further fractionated. The cells are then grown in primary culture. Immortalization may be accomplished by growing and passaging the cells through the cell crisis period, at which point the majority of cultured cells die; the resulting cells are immortal. Alternative methods for producing immortal T-cells and B-cells are described *supra.*

HCV replication in the HCV infected cell lines prepared as described *supra* may be detected by a variety of assays. For example, viral assays may depend upon the cytopathic or transforming characteristics of the virus. Cytopathic viruses may be assayed by their antimetabolic effects in microtitration plates or by the formation of characteristic plaques in monolayers of the appropriate susceptible host cell. A viral suspension is serially diluted and aded to monolayer culture plates. The number of plaques forming at the limiting dilution is taken as equivalent to the number of infectious particles in the supernatant medium, allowing the concentration of virus in the initial sample to be calculated. Characterization of the virus may be performed with specific antisera, measuring the cytopathic effect of the virus, by immunoassay, or by the cPCR/HCV assay for HCV RNA, described *supra*.

Transforming viruses may be assayed by, for example, the selective growth of transformed clones in suspension, or by looking for transformation foci in monolayer cultures.

In the event that the virus of interest is exhibits neither a detectable cytopathic nor a transforming effect, it may be detected in the infected cell preparation or infected cell lysate by a number of techniques, including, for example, immunoassay using a labeled antibody(s) which is specific for the virus, immunoassay of polypeptides labeled during replication using anti-HCV antibodies, and the detection of viral nucleic acids, for example, an enriched population of negative strands complementary to viral positive strands, or of viral nucleic acids labelled during an *in vitro* viral synthesis period.

### II.C. Immunoassays Using Antigens from HCV Infected Cells

The antigens from HCV infected cells which react immunologically with serum containing HCV antibodies are useful in immunoassays to detect presence of HCV antibodies in biological samples. The HCV antigens can take the form of, inter alia, purified or partially purified virus particles or viral polypeptides, disrupted virus, or infected cells or cell lysate therefrom. The production of the various forms of viral antigen from tissue culture sources is well-known in the art. Design of the immunoassays is subject to a great deal of variation, and a variety of these are known in the art. For example, an immunoassay may use a combination of viral epitopes derived from the same or from different viral polypeptides. It may use, for example, one or more viral antigens isolated from the HCV infected cell or from isolated HCV. Alternatively, fixed cells directly from culture may be used if viral epitopes are expressed internally or externally, or alternatively, cellular lysates may be used. It may also use, for example, whole disrupted virions. General methods for preparing disrupted virions are known in the art.

Immunoassay protocols may vary. Protocols may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

A suitable immunoassay may utilize disrupted virons in an ELISA format. The enzyme-linked immunosorbent assay (ELISA) method depends upon conjugation of an enzyme to either an antigen or an antibody, and uses the bound enzyme activity as a quantitative label. To measure antibody, the known antigen is fixed to a solid phase, (e.g., a microplate or plastic cup), incubated with anti-immunoglobulin labeled with enzyme, and washed again. Enzymes suitable for labeling are known in the art, and include, for example, horseradish peroxidase. Enzyme activity bound to the solid phase is measured by adding the specific substrate, and determining product formation or substrate utilization colorimetrically. The enzyme activity bound is a direct function of the amount of antibody bound.

To measure antigen, a known specific antibody is fixed to the solid phase, the test material containing antigen is added, after an incubation the solid phase is washed, and a second enzyme-labeled antibody is added. After washing, substrate is added, and enzyme activity is estimated colorimetrically, and related to antigen concentration.

Thus, for example, to detect anti-HCV antibodies using an ELISA format, isolated HCV, solubilized, e.g., with detergent, may be-used to coat appropriate microtiter plates. After the viral antigens are bound, another protein is also used to coat the plates, to prevent nonspecific binding of antibodies. The biological sample to be tested for anti-HCV antibodies is added to the plates, and the formation of HCV antibody-HCV antigen complexes is detected.

Another suitable immunoassay may utilize gel electrophoresis of HCV lysates, followed by Western blotting with the biological sample to be tested for anti-HCV antibodies. The formation of HCV antibody-HCV antigen complexes is detected using techniques known in the art.

Kits suitable for immunodiagnosis and containing the appropriate labeled reagents are constructed by packaging the appropriate materials, including the antigens derived from the HCV infected cells in suitable containers, along with the remaining reagents and materials required for the conduct of the assay, as well as a suitable set of assay instructions.

### II.D. Use of Infectable Cells in Screening for Antiviral Agents

The cells described herein which are capable of replicating HCV, may be used to screen for antiviral agents which inhibit HCV replication, and particularly for those agents which preferentially allow cell growth and multiplication while inhibiting viral replication. The screening methods which can be used are those which examine the inhibition of virus replication in the presence of the putative antiviral agent. Generally, the antiviral agents are tested at a variety of concentrations, for their effect on preventing viral replication in a cell culture system which supports viral replication, and then for an inhibition of infectivity or of viral pathogenicity (and a low level of toxicity) in an animal model system.

Included within antiviral agents are antibodies which block viral infection (neutralizing antibodies). The ability of anti-HCV antibodies to prevent the infection of the cells *in vitro* can be determined using the assays described herein to determine whether viral replication is inhibited by the presence of the putative anti-HCV antibodies. For example also, virus produced in infected cells may also be used as a source of antigens to determine whether antibodies are HCV specific.

### III. Examples

### III.A. Detection of HCV RNA in Mononuclear Cells From Individuals with NANBH

The detection of HCV RNA in mononuclear cells from blood of individuals with chronic NANBH was accomplished using essentially the cPCR/HCV assay described below.

Blood obtained from 5 individuals with chronic NANBH and which tested positive for anti-HCV antibodies using an HCV-C100 antigen immunoassay, and from 5 control individuals (reportedly free of NANBH, and which was negative in the immunoassay) was examined for the presence of HCV RNA in the mononuclear fraction. A mononucleocyte fraction from the samples was obtained by sedimentation through Ficoll-Paque (Pharmacia), following the manufacturer's directions.

The cPCR/HCV assay used in these studies were performed utilizing the following methods for the preparation of RNA, the reverse transcription of the RNA into cDNA, the amplification of specific segments of the cDNA by PCR, and the analysis of the PCR products.

Total RNA was isolated from the mononucleocyte fraction. The cDNA used as a template for the PCR reaction was prepared utilizing the designated samples for preparation of the corresponding cDNAs. Each RNA sample (corresponding to cells in 10 microliters of blood) was incubated in a 25 microliter reaction containing 1 micromolar of each primer, 1 millimolar of each deoxyribonucleotide triphosphate (dNTP), 50 millimolar Tris-HCL, pH 8.3, 5 millimolar MgCl₂, 5 millimolar dithiothreitol (DTT), 73 millimolar KCl, 40 units of RNase inhibitor (RNASIN), and 5 units of AMV reverse transcriptase. The incubation was for 60 minutes at 37°C. Following cDNA synthesis, the reactions were diluted with 50 microliters of deionized water (DIW), boiled for 10 minutes, and cooled on ice.

Amplification of a segment of the HCV cDNA was performed utilizing two synthetic 16-mer oligonucleotide primers whose sequences were derived from HCV cDNA clones 36 (anti-sense) and 37b (sense). The sequence of the primer from clone 36 was: The sequence of the primer from clone 37b was: The primers were used at a final concentration of 1 micromolar each. In order to amplify the segment of HCV cDNA which is flanked by the primers, the cDNA samples were incubated with 0.1 microgram of RNAse A and the PCR reactants of the Perkin Elmer Cetus PCR kit (N801-0043 or N801-0055) according to the manufacturer's instructions. The PCR reaction was performed for either 30 cycles or 60 cycles in a Perkin Elmer Cetus DNA thermal cycler. Each cycle consisted of a 1 minute denaturation step at 94°C, an annealing step of 2 minutes at 37°C, and an extension step of 3 minutes at 72°C. However, the extension step in the final cycle (30 or 60) was 7 minutes rather than 3 minutes. After amplification the samples were extracted with an equal volume of phenol: chloroform (1:1), followed by extraction with an equal volume of chloroform, and then the samples were precipitated with ethanol containing 0.2 M sodium acetate.

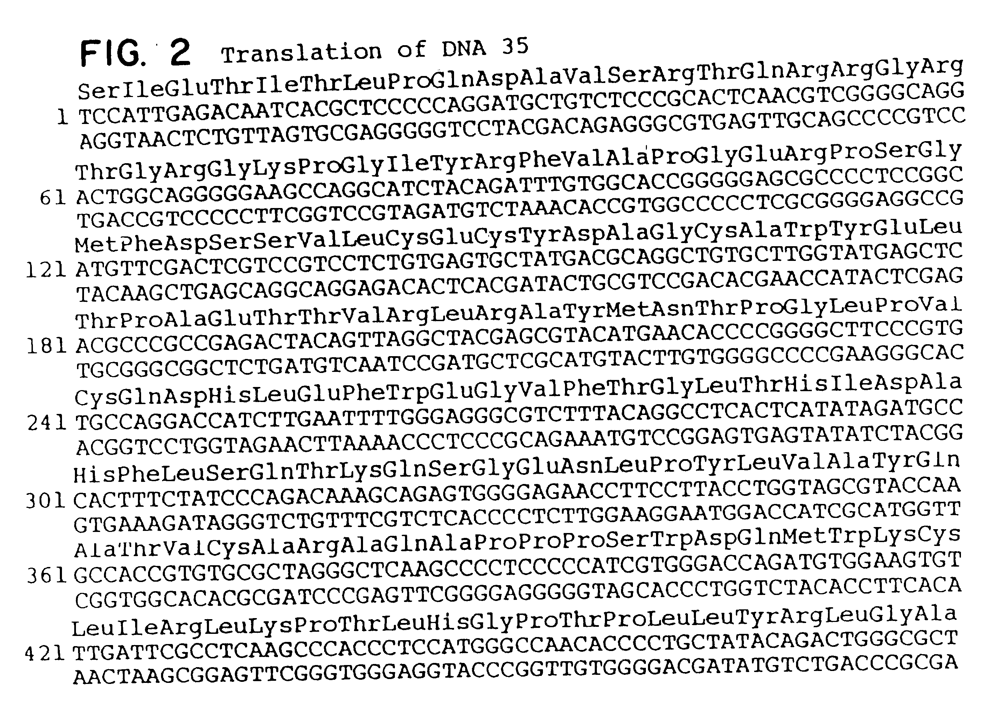

The cPCR products were analyzed as follows: The products were subjected to electrophoresis on 1.8% alkaline agarose gels according to Murakawa et al. (1988), and transferred onto Zeta Probe paper (BioRad Corp.) by blotting gels overnight in 0.4 M NaOH. The blots were neutralized in 2 × SSC (1 × SSC contains 0.15 M NaCl, 0.015 M sodium citrate), prehybridized in 0.3 M NaCl, 15 mM sodium phosphage buffer, pH 6.8, 15 mM EDTA, 1.0% SDS, 0.5% nonfat milk (Carnation Co.), and 0.5 mg/ml sonicated denatured salmon sperm DNA. The blots to be analyzed for HCV cDNA fragments were hybridized to a ³²P-labeled probe generated by nick translation of the HCV cDNA insert sequence in clone 35, shown in Figure 2. After hybridization, the blots were washed in 0.1 × SSC (1 × SSC contains 0.15M NaCl, 0.01M Na citrate) at 65°C, dried, and autoradiographed. The expected product size is 586 nucleotides in length; products which hybridized with the probe and migrated in the gels in this size range were scored as positive for viral RNA.

As a control, cPCR primers designed to amplify α1-antitrypsin mRNA was performed to verify the presence of RNA in each sample analyzed. The coding region of the α1-antitrypsin gene is described in Rosenberg et al. (1984). Synthetic 16-mer oligonucleotide primers designed to amplify a 365 nucleotide fragment of the coding region of the α1-antitrypsin gene were derived from nucleotides 22-37 (sense) and nucleotides 372-387 (anti-sense). The PCR products were detected using a ³²P nick-translated probe which lies between, and not including, the cDNA/PCR primer sequences.

Due to the extreme sensitivity of the PCR reaction, all samples were run a minimum of three times. All false positive signals were eliminated when the following precautions were taken: 1) eliminating aerosols by using screw capped tubes with rubber O-ring seals; 2) pipetting with Ranin Microman positive displacement pipetters with disposable pistons/capillaries; and 3) selecting the oligonucleotide sequences for the cDNA and PCR primers from two non-contiguous cDNA clones.

The results showed that mononucleocytes from 5 of 5 HCV anti-C100 antibody positive samples were positive for HCV RNA in the cPCR HCV assay. All of the controls were negative in the same assay.

### III.B. Detection of HCV RNA Minus Strands in Mononucleocytes from HCV Infected Individuals

The detection of minus strands in peripheral blood mononucleocytes was accomplished using the cPCR/HCV assay essentially as described in Section III.A., except that the cDNA was synthesized using either the 16mer primer from clone 36 (i.e., primer 36/16A), described in Section III.A. to prime the plus strand of HCV RNA, or the complement of the 16-mer (i.e., primer 36/16B), to prime the negative strand. In order to carry out the subsequent PCR reaction, the cDNA reaction mixture was boiled and treated with RNase prior to the addition of the second primer.

The results of the cPCR/HCV assay showed that 5 of the HCV anti-C100 antibody samples were positive for HCV negative strands; of these five samples, two showed high levels of HCV RNA negative strands. All 5 of these samples were about equally positive for the plus strands of HCV RNA. Of the five control samples, all were negative for both plus and minus strands of HCV RNA.

As an additional control, plasma samples from three of the HCV positive individuals were also analyzed. The signal obtained from the positive strands were at least 5 to 10 times stronger than was the signal for the negative strands. Thus, the plasma samples were strongly positive for the plus strands, and only weakly positive to negative for the minus strands.

In HCV, which is Flavi-like, the viral genome is positive stranded RNA. The discovery of the great enrichment of negative strands of HCV RNA in monocytes relative to that in plasma, suggests that replicative intermediates of the viral genome are present in these cells.

### IV. Commercial Utility

HCV infected cell systems, which are infected and/or replicated *in vitro*, including HCV infected cell lines, serve as a source of viral antigens which are useful in the detection of HCV antibodies, and hence in the diagnosis and the screening of biological materials (including blood) for HCV. These antigens also may have use in the preparation of vaccines for the treatment (prophylactic and/or therapeutic) of viral infections.

In addition, these cell systems are useful in the screening of anti-viral agents directed at preventing HCV replication, including antibodies which can neutralize infection or mediate antibody-dependent cellular cytotoxicity (ADCC).

While the invention has been described above by way of illustration, the present invention is not limited thereto and its scope is to be defined by the appended claims and the equivalents thereof.

## Claims (Claims for the following Contracting State(s): AT, BE, CH, LI, DE, DK, FR, GB, IT, LU, NL, SE)

1. A cell line infected with hepatitis C virus (HCV), wherein the cells in the cell line are B lymphocytes or T lymphocytes and are capable of replicating HCV.

2. The cell line of claim 1 wherein the cells in the cell line are B lymphocytes.

3. The cell line of claim 1 wherein the cells in the cell line are T lymphocytes.

4. The cell line of claim 3, wherein the cells are derived by infection of a cell from the cell line MOLT-4.

5. A method of preparing a composition for the detection of an anti-HCV antibody which comprises: purifying or partially purifying HCV virus particles or polypeptides from a cell line according to any one of claim 1 to 4; and fixing said particles or polypeptides to a solid phase.

6. A method according to claim 5 wherein HCV particles are solubilized with a detergent.

7. A method according to claim 5 or 6 wherein the composition for the detection of anti-HCV antibodies is packaged in a suitable container to provide a test kit.

8. A method for screening for an agent which inhibits HCV replication comprising:
(a) providing a composition comprised of a putative anti-viral agent;
(b) providing a cell line capable of replicating HCV;
(c) providing a composition comprised of infective HCV;
(d) incubating the compositions of (a) and (c) with the cell line of (b) under conditions which would, in the absence of (a) allow infection of and replication of HCV in the cell line; and
(e) detecting an inhibition of viral replication after the incubating in (d), wherein the cells in the cell line of (b) are B lymphocytes or T lymphocytes.

## Claims (Claims for the following Contracting State(s): ES, GR)

1. A method of preparing a composition for the detection of an anti-HCV antibody which comprises: purifying or partially purifying HCV virus particles or polypeptides from a cell line infected with hepatitis C virus (HCV), wherein the cells in the cell line are B lymphocytes or T lymphocytes and are capable of replicating HCV; and fixing said particles or polypeptides to a solid phase.

2. The method of claim 1 wherein the cells in the cell line are B lymphocytes.

3. The method of claim 1 wherein the cells in the cell line are T lymphocytes.

4. The method of claim 3, wherein the cells are derived by infection of a cell from the cell line MOLT-4.

5. A method according to any one of claims 1 to 4 wherein HCV particles are solubilized with a detergent.

6. A method according to any one of claims 1 to 5 wherein the composition for the detection of anti-HCV antibodies is packaged in a suitable container to provide a test kit.

7. A method for screening for an agent which inhibits HCV replication comprising:
(a) providing a composition comprised of a putative anti-viral agent;
(b) providing a cell line capable of replicating HCV;
(c) providing a composition comprised of infective HCV;
(d) incubating the compositions of (a) and (c) with the cell line of (b) under conditions which would, in the absence of (a) allow infection of and replication of HCV in the cell line; and
(e) detecting an inhibition of viral replication after the incubating in (d), wherein the cells in the cell line of (b) are B lymphocytes or T lymphocytes.

8. A cell line infected with hepatitis C virus (HCV), wherein the cells in the cell line are B lymphocytes or T lymphocytes and are capable of replicating HCV.

9. The cell line of claim 8 wherein the cells in the cell line are B lymphocytes.

10. The cell line of claim 8 wherein the cells in the cell line are T lymphocytes.

11. The cell line of claim 10, wherein the cells are derived by infection of a cell from the cell line MOLT-4.

## Patentansprüche (Patentansprüche für folgende(n) Vertragsstaat(en): AT, BE, CH, LI, DE, DK, FR, GB, IT, LU, NL, SE)

1. Zellinie, infiziert mit Hepatitis C-Virus (HCV), dadurch **gekennzeichnet**, daß die Zellen der Zellinie B-Lymphozyten oder T-Lymphozyten sind und in der Lage sind, HCV zu replizieren.

2. Zellinie nach Anspruch 1, dadurch **gekennzeichnet**, daß die Zellen in der Zellinie B-Lymphozyten sind.

3. Zellinie nach Anspruch 1, dadurch **gekennzeichnet**, daß die Zellen in der Zellinie T-Lymphozyten sind.

4. Zellinie nach Anspruch 3, dadurch **gekennzeichnet**, daß die Zellen durch Infektion einer Zelle von der Zellinie MOLT-4 abgeleitet sind.

5. Verfahren zur Herstellung einer Zusammensetzung zum Nachweis eines Anti-HCV-Antikörpers, umfassend: Reinigen oder teilweises Reinigen von HCV-Viruspartikeln oder Polypeptiden aus eine Zellinie nach einem der Ansprüche 1 bis 4; und Fixieren der Partikel oder Polypeptide an eine feste Phase.

6. Verfahren nach Anspruch 5, dadurch **gekennzeichnet**, daß die HCV-Partikel in einem Detergens solubilisiert werden.

7. Verfahren nach Anspruch 5 oder 6, dadurch **gekennzeichnet**, daß die Zusammensetzung zum Nachweis von Anti-HCV-Antikörpern in einem geeigneten Behälter verpackt ist, um einen Testkit bereitzustellen.

8. Verfahren zum Durchmustern eines Agens, welches die HCV-Replikation inhibiert, umfassend:
(a) Bereitstellung einer Zusammensetzung, umfassend ein mögliches antivirales Agens;
(b) Bereitstellung einer Zellinie, die in der Lage ist, HCV zu replizieren;
(c) Bereitstellung einer Zusammensetzung, welche infektiöses HCV umfaßt;
(d) Inkubation der Zusammensetzungen (a) und (c) mit der Zellinie (b) unter Bedingungen, die in Abwesenheit von (a) die Infektion der Zellinie und die Replikation von HCV in der Zellinie ermöglichen würden; und
(e) Nachweis einer Inhibition der viralen Replikation nach Inkubation in (d), wobei die Zellen in der Zellinie (b) B-Lymphozyten oder T-Lymphozyten sind.

## Patentansprüche (Patentansprüche für folgende(n) Vertragsstaat(en): ES, GR)

1. Verfahren zur Herstellung einer Zusammensetzung zum Nachweis eines Anti-HCV-Antikörpers, umfassend: Reinigen oder teilweises Reinigen von HCV-Viruspartikeln oder Polypeptiden aus einer Zellinie, die mit Hepatitis C-Virus (HCV) infiziert ist, dadurch **gekennzeichnet**, daß die Zellen in der Zellinie B-Lymphozyten oder T-Lymphozyten sind und in der Lage sind, HCV zu replizieren; und Fixieren der Partikel oder Polypeptide an einer festen Phase.

2. Verfahren nach Anspruch 1, dadurch **gekennzeichnet**, daß die Zellen in der Zellinie B-Lymphozyten sind.

3. Verfahren nach Anspruch 1, dadurch **gekennzeichnet**, daß die Zellen in der Zellinie T-Lymphozyten sind.

4. Verfahren nach Anspruch 3, dadurch **gekennzeichnet**, daß die Zellen durch Infektion einer Zelle aus der Zellinie MOLT-4 abgeleitet sind.

5. Verfahren nach einem der Ansprüche 1 bis 4, dadurch **gekennzeichnet**, daß die HCV-Partikel mit einem Detergens solubilisiert werden.

6. Verfahren nach einem der Ansprüche 1 bis 5, dadurch **gekennzeichnet**, daß die Zusammensetzung zum Nachweis von Anti-HCV-Antikörpern in einem geeigneten Behältnis verpackt ist, um einen Testkit bereitzustellen.

7. Verfahren zum Durchmustern eines Agens, welches die HCV-Replikation inhibiert, umfassend:
(a) Bereitstellung einer Zusammensetzung, umfassend ein mögliches antivirales Agens;
(b) Bereitstellung einer Zellinie, die in der Lage ist, HCV zu replizieren;
(c) Bereitstellung einer Zusammensetzung, welche infektiöses HCV umfaßt;
(d) Inkubation der Zusammensetzungen (a) und (c) mit der Zellinie (b) unter Bedingungen, die in Abwesenheit von (a) die Infektion der Zellinie und die Replikation von HCV in der Zellinie ermöglichen würden; und
(e) Nachweis einer Inhibition der viralen Replikation nach Inkubation in (d), wobei die Zellen in der Zellinie (b) B-Lymphozyten oder T-Lymphozyten sind.

8. Zellinie, infiziert mit Hepatitis C-Virus (HCV), dadurch **gekennzeichnet**, daß die Zellen in der Zellinie B-Lymphozyten oder T-Lymphozyten sind und in der Lage sind, HCV zu replizieren.

9. Zellinie nach Anspruch 8, dadurch **gekennzeichnet**, daß die Zellen in der Zellinie B-Lymphozyten sind.

10. Zellinie nach Anspruch 8, dadurch **gekennzeichnet**, daß die Zellen in der Zellinie T-Lymphozyten sind.

11. Zellinie nach Anspruch 10, dadurch **gekennzeichnet**, daß die Zellen durch Infektion einer Zelle aus der Zellinie MOLT-4 abgeleitet sind.

## Revendications (Revendications pour l'(les) Etat(s) contractant(s) suivant(s): AT, BE, CH, LI, DE, DK, FR, GB, IT, LU, NL, SE)

1. Lignée cellulaire infectée avec le virus de l'hépatite C (HCV), dans laquelle les cellules dans la lignée cellulaire sont des lymphocytes B ou des lymphocytes T et sont capables de répliquer HCV.

2. Lignée cellulaire de la revendication 1 dans laquelle les cellules dans la lignée cellulaire sont des lymphocytes B.

3. Lignée cellulaire de la revendication 1 dans laquelle les cellules dans la lignée cellulaire sont des lymphocytes T.

4. Lignée cellulaire de la revendication 3 dans laquelle les cellules sont dérivées par infection d'une cellule provenant de la lignée cellulaire MOLT-4.

5. Méthode de préparation d'une composition pour la détection d'un anticorps anti-HCV qui comprend les étapes consistant : à purifier ou à purifier de façon partielle des particules de virus HCV ou des polypeptides provenant d'une lignée cellulaire selon l'une quelconque des revendications 1 à 4; et à fixer lesdites particules ou lesdits polypeptides sur une phase solide.

6. Méthode selon la revendication 5 dans laquelle les particules d'HCV sont solubilisées avec un détergent.

7. Méthode selon la revendication 5 ou 6 dans laquelle la composition pour la détection d'anticorps anti-HCV est conditionnée dans un récipient approprié pour fournir un kit de test.

8. Méthode pour cribler un agent qui inhibe la réplication d'HCV comprenant les étapes consistant :
(a) à fournir une composition comprenant un agent anti-viral putatif ;
(b) à fournir une lignée cellulaire capable de répliquer HCV ;
(c) à fournir une composition comprenant du HCV infectieux ;
(d) à faire incuber les compositions de (a) et (c) avec la lignée cellulaire de (b) dans des conditions qui, en l'absence de (a), permettraient l'infection et la réplication de HCV dans la lignée cellulaire ; et
(e) à détecter une inhibition de la réplication virale après l'incubation dans (d), dans laquelle les cellules dans la lignée cellulaire de (b) sont des lymphocytes B ou des lymphocytes T.

## Revendications (Revendications pour l'(les) Etat(s) contractant(s) suivant(s): ES, GR)

1. Méthode de préparation d'une composition pour la détection d'un anticorps anti-HCV qui comprend les étapes consistant : à purifier ou à purifier de façon partielle des particules de virus HCV ou des polypeptides provenant d'une lignée cellulaire infectée avec le virus de l'hépatite C (HCV), dans laquelle les cellules dans la lignée cellulaire sont des lymphocytes B ou des lymphocytes T et sont capables de réplication d'HCV; et à fixer lesdites particules ou polypeptides sur une phase solide.

2. Méthode de la revendication 1 dans laquelle les cellules dans la lignée cellulaire sont des lymphocytes B.

3. Méthode de la revendication 1 dans laquelle les cellules dans la lignée cellulaire sont des lymphocytes T.

4. Méthode de la revendication 3, dans laquelle les cellules sont dérivées par infection d'une cellule provenant de la lignée cellulaire MOLT-4.

5. Méthode selon l'une quelconque des revendications 1 à 4 dans laquelle les particules d'HCV sont solubilisées avec un détergent.

6. Méthode selon l'une quelconque des revendications 1 à 5 dans laquelle la composition pour la détection d'anticorps anti-HCV est conditionnée dans un récipient approprié pour fournir un kit de test.

7. Méthode pour l'âge d'un agent qui inhibe la réplication d'HCV comprenant les étapes consistant :
(a) à fournir une composition contenant un agent anti-viral putatif;
(b) à fournir une lignée cellulaire capable de réplication HCV;
(c) à fournir une composition comprenant HCV infectieux;
(d) à incuber les compositions de (a) et (c) avec la lignée cellulaire (b) dans des conditions qui permettraient, en l'absence de (a) de permettre l'infection de et la réplication de HCV dans la lignée cellulaire; et
(e) à détecter une inhibition de la réplication virale après l'incubation dans (d), dans laquelle les cellules dans la lignée cellulaire (b) sont des lymphocytes B ou des lymphocytes T.

8. Lignée cellulaire infectée avec le virus de l'hépatite C (HCV), dans laquelle les cellules dans la lignée cellulaire sont des lymphocytes B ou des lymphocytes T et sont capables de réplication d'HCV.

9. Lignée cellulaire de la revendication 8 dans laquelle les cellules dans la lignée cellulaire sont des lymphocytes B.

10. Lignée cellulaire de la revendication 8 dans laquelle les cellules dans la lignée cellulaire sont des lymphocytes T.

11. Lignée cellulaire de la revendication 10 dans laquelle les cellules sont dérivées par infection d'une cellule provenant de la lignée cellulaire MOLT-4.
